(19) Europäisches Patentamt
European Patent Office
Office européen des brevets

(11) EP 1 370 214 B1

(12) EUROPÄISCHE PATENTSCHRIFT

(45) Veröffentlichungstag und Bekanntmachung des Hinweises auf die Patenterteilung:
**22.11.2006 Patentblatt 2006/47**

(21) Anmeldenummer: **02704756.2**

(22) Anmeldetag: **14.03.2002**

(51) Int Cl.:
***A61K 8/04*** *(2006.01)* ***A61Q 1/00*** *(2006.01)*
***A61Q 17/04*** *(2006.01)* ***A61Q 19/00*** *(2006.01)*

(86) Internationale Anmeldenummer:
**PCT/EP2002/002827**

(87) Internationale Veröffentlichungsnummer:
**WO 2002/074253 (26.09.2002 Gazette 2002/39)**

(54) **SELBSTSCHÄUMENDE ODER SCHAUMFÖRMIGE ZUBEREITUNGEN MIT ANORGANISCHEN GELBILDNERN UND ORGANISCHEN HYDROKOLLOIDEN**

SELF-FOAMING OR MOUSSE-TYPE PREPARATIONS COMPRISING INORGANIC GEL-FORMING AGENTS AND ORGANIC HYDROCOLLOIDS

PREPARATIONS AUTO-MOUSSANTES OU SOUS FORME DE MOUSSES COMPORTANT DES GELIFIANTS INORGANIQUES ET DES HYDROCOLLOIDES ORGANIQUES

(84) Benannte Vertragsstaaten:
**AT BE CH CY DE DK ES FI FR GB GR IE IT LI LU MC NL PT SE TR**

(30) Priorität: **15.03.2001 DE 10113053**

(43) Veröffentlichungstag der Anmeldung:
**17.12.2003 Patentblatt 2003/51**

(73) Patentinhaber: **Beiersdorf AG**
**20245 Hamburg (DE)**

(72) Erfinder:
- **BLECKMANN, Andreas**
**22926 Ahrensburg (DE)**
- **KRÖPKE, Rainer**
**22869 Schenefeld (DE)**
- **RIEDEL, Heidi**
**22529 Hamburg (DE)**

(56) Entgegenhaltungen:
**EP-A- 0 257 336**
**EP-A- 1 010 421**
**EP-A- 1 046 387**
**US-A- 4 364 515**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft selbstschäumende und/oder schaumförmige kosmetische und dermatologische Zubereitungen, insbesondere hautpflegende kosmetische und dermatologische Zubereitungen.

Schäume bzw. schaumförmige Zubereitungen gehören zu den dispersen Systemen.

Das bei weitem wichtigste und bekannteste disperse System stellen Emulsionen dar. Emulsionen sind Zwei- oder Mehrphasensysteme von zwei oder mehr ineinander nicht oder nur wenig löslichen Flüssigkeiten. Die Flüssigkeiten (rein oder als Lösungen) liegen in einer Emulsion in einer mehr oder weniger feinen Verteilung vor, die im allgemeinen nur begrenzt stabil ist.

Schäume sind Gebilde aus gasgefüllten, kugel- oder polyederförmigen Zellen, welche durch flüssige, halbflüssige, hochviskose oder feste Zellstege begrenzt werden. Die Zellstege, verbunden über sogenannte Knotenpunkte, bilden ein zusammenhängendes Gerüst. Zwischen den Zellstegen spannen sich die Schaumlamellen (geschlossenzelliger Schaum). Werden die Schaumlamellen zerstört oder fließen sie am Ende der Schaumbildung in die Zellstege zurück, erhält man einen offenzelligen Schaum. Auch Schäume sind thermodynamisch instabil, da durch Verkleinerung der Oberfläche Oberflächenenergie gewonnen werden kann. Die Stabilität und damit die Existenz eines Schaums ist somit davon abhängig, wieweit es gelingt, seine Selbstzerstörung zu verhindern.

Kosmetische Schäume sind in der Regel dispergierte Systeme aus Flüssigkeiten und Gasen, wobei die Flüssigkeit das Dispergiermittel und das Gas die dispergierte Substanz darstellen. Schäume aus niedrigviskosen Flüssigkeiten werden temporär durch oberflächenaktive Substanzen (Tenside, Schaumstabilisatoren) stabilisiert. Solche Tensidschäume haben aufgrund ihrer großen inneren Oberfläche ein starkes Adsorptionsvermögen, welches beispielsweise bei Reinigungs- und Waschvorgängen ausgenutzt wird. Dementsprechend finden kosmetische Schäume insbesondere in den Bereichen der Reinigung, beispielsweise als Rasierschaum, und der Haarpflege Verwendung.

Zur Erzeugung von Schaum wird Gas in geeignete Flüssigkeiten eingeblasen, oder man erreicht die Schaumbildung durch heftiges Schlagen, Schütteln, Verspritzen oder Rühren der Flüssigkeit in der betreffenden Gasatmosphäre, vorausgesetzt, daß die Flüssigkeiten geeignete Tenside oder andere grenzflächenaktive Stoffe (sogenannte Schaumbildner) enthalten, die außer Grenzflächenaktivität auch ein gewisses Filmbildungsvermögen besitzen.

Kosmetische Schäume haben gegenüber anderen kosmetischen Zubereitungen den Vorteil, daß sie eine feine Verteilung von Wirkstoffen auf der Haut erlauben. Allerdings sind kosmetische Schäume in der Regel nur durch Verwendung besonderer Tenside, welche darüberhinaus oft wenig hautverträglich sind, zu erreichen.

Ein weiterer Nachteil des Standes der Technik ist es, daß derartige Schäume nur wenig stabil sind, weshalb sie üblicherweise innerhalb von etwa 24 Stunden zusammenfallen. Eine Anforderung an kosmetische Zubereitungen ist aber, daß diese eine möglichst jahrelange Stabilität besitzen. Diesem Problem wird im allgemeinen dadurch Rechnung getragen, daß der Verbraucher den eigentlichen Schaum erst bei der Anwendung mit Hilfe eines geeigneten Sprühsystems selbst erzeugt, wozu beispielsweise Sprühdosen verwendet werden können, in denen ein verflüssigtes Druckgas als Treibgas dient. Beim Öffnen des Druckventils entweicht das Treibmittel-Flüssigkeitsgemisch durch eine feine Düse, das Treibmittel verdampft und hinterläßt einen Schaum.

Auch nachschäumende kosmetische Zubereitungen sind an sich bekannt. Sie werden zunächst in fließförmiger Form aus einem Aerosolbehälter auf die Haut aufgetragen und entwickeln nach kurzer Verzögerung erst dort unter dem Einfluß des enthaltenen Nachschäummittels den eigentlichen Schaum, beispielsweise einen Rasierschaum. Nachschäumende Zubereitungen liegen oft in speziellen Ausführungsformen wie etwa nachschäumenden Rasiergelen oder dergleichen vor.

Allerdings kennt der Stand der Technik keinerlei kosmetische oder dermatologische Zubereitungen, welche bereits bei der Herstellung aufgeschäumt werden könnten und dennoch eine genügend hohe Stabilität aufweisen, um in üblicher Weise verpackt, gelagert und in den Handel gebracht zu werden.

Eine Aufgabe der vorliegenden Erfindung war also, den Stand der Technik zu bereichern und kosmetische oder dermatologische selbstschäumende und/oder schaumförmige Zubereitungen zur Verfügung zu stellen, die die Nachteile des Standes der Technik nicht aufweisen.

Die Deutsche Offenlegungsschrift DE 197 54 659 offenbart, daß Kohlendioxid ein geeigneter Wirkstoff zur Stabilisierung oder Erhöhung der epidermalen Ceramidsyntheserate ist, welcher der Stärkung der Permeabilitätsbarriere, der Verminderung des transepidermalen Wasserverlusts und der Steigerung der relativen Hautfeuchtigkeit dienen kann. Zur Behandlung der Haut wird das $CO_2$ beispielsweise in Wasser gelöst, mit welchem anschließend die Haut gespült wird. Allerdings kennt der Stand der Technik bislang keinerlei kosmetische oder dermatologische Grundlagen, in die ein gasförmiger Wirkstoff in ausreichender, d. h. wirksamer Konzentration eingearbeitet werden könnte.

Eine weitere Aufgabe der vorliegenden Erfindung war es also, kosmetische oder dermatologische Grundlagen zu finden, in die sich wirksame Mengen an gasförmigen Wirkstoffen einarbeiten lassen.

Es war überraschend und für den Fachmann nicht vorauszusehen, daß
selbstschäumende und/oder schaumförmige kosmetische oder dermatologische Zubereitungen, welche

I. ein Emulgatorsystem, welches aus

A. mindestens einem Emulgator A, gewählt aus der Gruppe der ganz-, teil- oder nichtneutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,

besteht,
II. bis zu 30 Gew.-% - bezogen auf das Gesamtgewicht der Zubereitung - einer Lipidphase,
III. 1 bis 90 Vol.%, bezogen auf das Gesamtvolumen der Zubereitung, mindestens eines Gases, gewählt aus der Gruppe Luft, Sauerstoff, Stickstoff, Helium, Argon, Lachgas ($N_2O$) und Kohlendioxid ($CO_2$)
IV. 0,01 - 10% Gew. % eines oder mehrerer Gelbildner ausgewählt aus der Gruppe der anorganischen Verdikkungsmitteln,
V. eine oder mehrere Substanzen, gewählt aus der Gruppe der organischen Hydrokolloide,

enthalten,
den Nachteilen des Standes der Technik abhelfen.

Nach dem bisherigen Stand der Technik sind schaumförmige kosmetische Emulsionen, die sich durch einen hohen Lufteintrag auszeichnen, ohne Treibgas nicht zu formulieren bzw. technisch herzustellen. Dieses gilt insbesondere für Systeme, die auf klassischen Emulgatoren und Gelbildnern basieren und durch Scherung (Rühren, Homogenisierung) einen Schaum mit einer außerordentlich hohen Stabilität entwickeln. Durch die Erfindung wird der Eintrag der Gase unterstützt sowie über eine längere Lagerdauer auch bei höheren Temperaturen (z.B. 40 °C) ein stabilisierender sowie deutlich nachschäumender Effekt erzielt, ohne nach dem Stand der Technik übliche Nachschäummittel wie z.B. durch Treibgase zu enthalten.

Hierdurch ist es erstmals möglich gegenüber dem bisherigen Stand der Technik Rezepturen mit einer herausragenden, neuartigen kosmetischen Wirkleistung und mit außerordentlich hohem Gasvolumen (Luft und/oder andere Gase wie Sauerstoff, Kohlendioxid, Stickstoff, Helium, Argon u.a.) über lange Lagerdauer bei hohen Temperaturen stabil zu generieren. Gleichzeitig zeichnen sie sich durch eine überdurchschnittliche gute Hautpflege sowie sehr guten sensorischen Eigenschaften aus.

Unter "selbstschäumend" bzw. "schaumförmig" ist im Sinne der vorliegenden Erfindung zu verstehen, daß die Gasbläschen (beliebig) verteilt in einer (oder mehreren) flüssigen Phase(n) vorliegen, wobei die Zubereitungen makroskopisch nicht notwendigerweise das Aussehen eines Schaumes haben müssen. Erfindungsgemäße selbstschäumende und/oder schaumförmige kosmetische oder dermatologische Zubereitungen können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße selbstschäumende und/oder schaumförmige Zubereitungen - insbesondere dann, wenn die Gasbläschen zu klein sind, um unter einem Lichtmikroskop erkannt zu werden - auch an der starken Volumenzunahme des Systems erkennbar.

Die erfindungsgemäßen Zubereitungen stellen in jeglicher Hinsicht überaus befriedigende Präparate dar. Es war insbesondere überraschend, daß die erfindungsgemäßen schaumförmigen Zubereitungen - auch bei einem ungewöhnlich hohen Gasvolumen - außerordentlich stabil sind. Dementsprechend eignen sie sich ganz besonders, um als Grundlage für Zubereitungsformen mit vielfältigen Anwendungszwecken zu dienen. Die erfindungsgemäßen Zubereitungen zeigen sehr gute sensorische Eigenschaften, wie beispielsweise die Verteilbarkeit auf der Haut oder das Einzugsvermögen in die Haut, und zeichnen sich darüberhinaus durch eine überdurchschnittlich gute Hautpflege aus.

Gegenstand der Erfindung ist ferner
die Verwendung selbstschäumender und/oder schaumförmiger kosmetischer oder dermatologischer Zubereitungen, welche

I. ein Emulgatorsystem, welches aus

A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht,

und

II. bis zu 30 Gew.-% einer Lipidphase, bezogen auf das Gesamtgewicht der Zubereitung

IV. 0,01 - 10% Gew. % eines oder mehrerer Gelbildner ausgewählt aus der Gruppe der anorganischen Verdickungsmitteln,

V. eine oder mehrere Substanzen, gewählt aus der Gruppe der organischen Hydrokolloide,

enthalten, als kosmetische oder dermatologische Grundlagen für gasförmige Wirkstoffe.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Butyloctanol, Butyldecanol, Hexyloctanol, Hexyldecanol, Octyldodecanol, Behenylalkohol ($C_{22}H_{45}OH$), Cetearylalkohol [eine Mischung aus Cetylalkohol ($C_{16}H_{33}OH$) und Stearylalkohol ($C_{18}H_{37}OH$)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 8 bis 13 Gew.-%, jeweils bezogen auf das Gesamtgewicht der Formulierung, zu wählen.

Besonders bevorzugt im Sinne der vorliegenden Erfindung ist es, wenn die Gasphase der Zubereitungen Kohlendioxid enthält bzw. ganz aus Kohlendioxid besteht. Es ist insbesondere vorteilhaft, wenn Kohlendioxid einen oder den Wirkstoff in den erfindungsgemäßen Zubereitungen darstellt.

Erfindungsgemäße Zusammensetzungen entwickeln sich bereits während ihrer Herstellung - beispielsweise während des Rührens oder bei der Homogenisierung - zu feinblasigen Schäumen. Erfindungsgemäß sind feinblasige, reichhaltige Schäume von hervorragender kosmetischer Eleganz erhältlich. Weiterhin sind erfindungsgemäß besonders gut hautverträgliche Zubereitungen erhältlich, wobei wertvolle Inhaltsstoffe besonders gut auf der Haut verteilt werden können.

Es ist gegebenenfalls vorteilhaft, wenngleich nicht notwendig, wenn die Formulierungen gemäß der vorliegenden Erfindung weitere Emulgatoren enthalten. Vorzugsweise sind solche Emulgatoren zu verwenden, welche zur Herstellung von W/O-Emulsionen geeignet sind, wobei diese sowohl einzeln als auch in beliebigen Kombinationen miteinander vorliegen können.

Vorteilhaft werden der oder die weiteren Emulgatoren aus der Gruppe gewählt, die die folgenden Verbindungen umfaßt:

Polyglyceryl-2-Dipolyhydroxystearat, PEG-30-Dipolyhydroxystearat, Cetyldimethiconcopolyol, Glykoldistearat, Glykoldilaurat, Diethylenglykoldilaurat, Sorbitantrioleat, Glykololeat, Glyceryldilaurat, Sorbitantristearat, Propylenglykolstearat, Propylenglykollaurat, Propylenglykoldistearat, Sucrosedistearat, PEG-3 Castor Oil, Pentaerythritylmonostearat, Pentaerythritylsesquioleat, Glyceryloleat, Glycerylstearat, Glyceryldiisostearat, Pentaerythritylmonooleat, Sorbitansesquioleat, Isostearyldiglycerylsuccinat, Glycerylcaprat, Palm Glycerides, Cholesterol, Lanolin, Glyceryloleat (mit 40 % Monoester), Polyglyceryl--2-Sesquiisostearat, Polyglyceryl-2-Sesquioleat, PEG-20 Sorbitan Beeswax, Sorbitanoleat, Sorbitanisostearat, Trioleylphosphat, Glyceryl Stearate und Ceteareth-20 (Teginacid von Th. Goldschmidt), Sorbitanstearat, PEG-7 Hydrogenated Castor Oil, PEG-5-Soyasterol, PEG-6 Sorbitan Beeswax, Glycerylstearat SE, Methylglucosesesquistearate, PEG-10 Hydrogenated Castor Oil, Sorbitanpalmitat, PEG-22/Dodecylglykol Copolymer, Polyglyceryl-2-PEG-4-Stearat, Sorbitanlaurat, PEG-4-Laurat, Polysorbat 61, Polysorbat 81, Polysorbat 65, Polysorbat 80, Triceteareth-4-Phosphat, Triceteareth-4 Phosphate und Sodium $C_{14-17}$ Alkyl Sec Sulfonat (Hostacerin CG von Hoechst), Glycerylstearat und PEG-100 Stearate (Arlacel 165 von ICI), Polysorbat 85, Trilaureth-4-Phosphat, PEG-35 Castor Oil, Sucrosestearat, Trioleth-8-Phosphat, $C_{12-15}$ Pareth-12, PEG-40

Hydrogenated Castor Oil, PEG-16 Soya Sterol, Polysorbat 80, Polysorbat 20, Polyglyceryl-3-methylglucose Distearat, PEG-40 Castor Oil, Natriumcetearylsulfat, Lecithin, Laureth-4-Phosphat, Propylenglykolstearat SE, PEG-25 Hydrogenated Castor Oil, PEG-54 Hydrogenated Castor Oil, Glycerylstearat SE, PEG-6 Caprylic/Capric Glyceride, Glyceryloleat und Propylenglykol, Glyceryllanolat, Polysorbat 60, Glycerylmyristat, Glycerylisostearat und Polyglyceryl-3 Oleat, Glyceryllaurat, PEG-40-Sorbitanperoleat, Laureth-4, Glycerinmonostearat, Isostearylglycerylether, Cetearyl Alcohol und Natriumcetearylsulfat, PEG-22-Dodecylglykolcopolymer, Polyglyceryl-2-PEG-4-Stearat, Pentaerythrithylisostearat, Polyglyceryl-3-Diisostearat, Sorbitanoleat und Hydrogenated Castor Oil und Cera alba und Stearinsäure, Natriumdihydroxycetylphosphat und Isopropylhydroxycetylether, Methylglucosesesquistearat, Methylglucosedioleat, Sorbitanoleat und PEG-2 Hydrogenated Castor Oil und Ozokerit und Hydrogenated Castor Oil, PEG-2 Hydrogenated Castor Oil, PEG-45-/Dodecylglykolcopolymer, Methoxy PEG-22-/Dodecylglykolcopolymer, Hydrogenated Coco Glycerides, Polyglyceryl-4-Isostearat, PEG-40-Sorbitanperoleat, PEG-40-Sorbitanperisostearat, PEG-8-Beeswax, Laurylmethiconcopolyol, Polyglyceryl-2-Laurat, Stearamidopropyl-PG-dimoniumchloridphosphat, PEG-7 Hydrogenated Castor Oil, Triethylcitrat, Glycerylstearatcitrat, Cetylphosphat, Polyglycerolmethylglucosedistearat, Poloxamer 101, Kaliumcetylphosphat, Glycerylisostearat, Polyglyceryl-3-Diisostearate.

Bevorzugt werden der oder die weiteren Emulgatoren im Sinne der vorliegenden Erfindung aus der Gruppe der hydrophilen Emulgatoren gewählt. Erfindungsgemäß besonders bevorzugt sind Mono-, Di-, Trifettsäureestern des Sorbitols.

Die Gesamtmenge der weiteren Emulgatoren wird erfindungsgemäß vorteilhaft kleiner als 5 Gew.-%, bezogen auf das Gesamtgewicht der Formulierung, gewählt.

Die Liste der genannten weiteren Emulgatoren, die im Sinne der vorliegenden Erfindung eingesetzt werden können, soll selbstverständlich nicht limitierend sein.

Besonders vorteilhafte selbstschäumende und/oder schaumförmige Zubereitungen im Sinne der vorliegenden Erfindung sind frei von Mono- oder Diglycerylfettsäureestern. Insbesondere bevorzugt sind erfindungsgemäße Zubereitungen, welche kein Glycerylstearat, Glycerylisostearat, Glyceryldiisostearat, Glyceryloleat, Glycerylpalmitat, Glycerylmyristat, Glyceryllanolat und/oder Glyceryllaurat enthalten.

Die Ölphase der erfindungsgemäßen Zubereitungen wird vorteilhaft gewählt aus der Gruppe der unpolaren Lipide mit einer Polarität $\geq$ 30 mN/m. Besonders vorteilhafte unpolare Lipide im Sinne der vorliegenden Erfindung sind die im folgenden aufgelisteten.

| Hersteller | Handelsname | INCI-Name | Polarität mN/m |
|---|---|---|---|
| Total SA | Ecolane 130 | Cycloparaffin | 49,1 |
| Neste PAO N.V. (Lief. Hansen & Rosenthal) | Nexbase 2006 FG | Polydecene | 46.7 |
| Chemische Fabrik Lehrte | Polysynlane | Hydrogenated Polyisobutene | 44.7 |
| Wacker | Wacker Silikonöl AK 50 | Polydimethylsiloxan | 46,5 |
| EC Erdölchemie (Lieferant Bayer AG) | Solvent ICH | Isohexadecane | 43.8 |
| DEA Mineralöl (Lief. Hansen & Rosenthal) Tudapetrol | Pionier 2076 | Mineral Oil | 43.7 |
| DEA Mineralöl (Lief. Hansen & Rosenthal) Tudapetrol | Pionier 6301 | Mineral Oil | 43.7 |
| Wacker | Wacker Silikonöl AK 35 | Polydimethylsiloxan | 42,4 |
| EC Erdölchemie GmbH | Isoeikosan | Isoeikosan | 41.9 |
| Wacker | Wacker Silikonöl AK 20 | Polydimethylsiloxan | 40,9 |
| Condea Chemie | Isofol 1212 Carbonat | | 40,3 |
| Gattefossé | Softcutol O | Ethoxydiglycol Oleate | 40,5 |
| Creaderm | Lipodermanol OL | Decyl Olivate | 40,3 |
| Henkel | Cetiol S | Dioctylcyclohexane | 39,0 |

(fortgesetzt)

| Hersteller | Handelsname | INCI-Name | Polarität mN/m |
|---|---|---|---|
| DEA Mineralöl (Lief. Hansen & Rosenthal) Tudapetrol | Pionier 2071 | Mineral Oil | 38.3 |
| WITCO BV | Hydrobrite 1000 PO | Paraffinum Liquidum | 37,6 |
| Goldschmidt | Tegosoft HP | Isocetyl Palmitate | 36,2 |
| Condea Chemie | Isofol Ester 1693 | | 33,5 |
| Condea Chemie | Isofol Ester 1260 | | 33,0 |
| Dow Corning | Dow Corning Fluid 245 | Cyclopentasiloxan | 32,3 |
| Unichema | Prisorine 2036 | Octyl Isostearate | 31.6 |
| Henkel Cognis | Cetiol CC | Dicaprylyl Carbonate | 31,7 |
| ALZO (ROVI) | Dermol 99 | Trimethylhexyl Isononanoate | 31,1 |
| ALZO (ROVI) | Dermol 89 | 2- Ethylhexyl Isononanoate | 31,0 |
| Unichema | Estol 1540 EHC | Octyl Cocoate | 30.0 |

Von den Kohlenwasserstoffen sind insbesondere Paraffinöl sowie weitere hydrierte Polyolefine wie hydriertes Polyisobutene, Squalan und Squalen vorteilhaft im Sinne der vorliegenden Erfindung zu verwenden.

Die Gehalt der Lipidphase wird vorteilhaft kleiner als 30 Gew.-% gewählt, bevorzugt zwischen 2,5 und 30 Gew.-%, insbesondere bevorzugt zwischen 5 und 15 Gew.-%, jeweils bezogen auf das Gesamtgewicht der Zubereitung. Es ist gegebenenfalls ferner vorteilhaft, wenngleich nicht zwingend, wenn die Lipidphase bis zu 40 Gew.-% - bezogen auf das Gesamtgewicht der Lipidphase - an polaren Lipiden (mit einer Polarität ≤ 20 mN/m) und/oder mittelpolaren Lipiden (mit einer Polarität von 20 bis 30 mN/m) enthält.

Besonders vorteilhafte polare Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

| Hersteller | Handelsname | INCI-Name | Polarität mN/m |
|---|---|---|---|
| Condea Chemie | Isofol 14 T | Butyl Decanol (+) Hexyl Octanol (+) Hexyl Decanol (+) Butyl Octanol | 19,8 |
| Lipochemicals INC. / USA (Induchem) | Lipovol MOS-130 | Tridecyl Stearate(+) Tridecyl Trimellitate(+) Dipentaerythrityl Hexacaprylate/Hexacaprate | 19,4 |
| | Ricinusoel | | 19,2 |
| CONDEA Chemie | Isofol Ester 0604 | | 19,1 |
| Huels CONDEA Chemie | Miglyol 840 | Propylene Glycol Dicaprylate/ Dicaprate | 18,7 |
| CONDEA Chemie | Isofol 12 | Butyl Octanol | 17,4 |
| Goldschmidt | Tegosoft SH | Stearyl Heptanoate | 17,8 |
| | Avocadooel | | 14,5 |
| Henkel Cognis | Cetiol B | Dibutyl Adipate | 14,3 |
| ALZO (ROVI) | Dermol 488 | PEG 2 Diethylenhexanoate | 10,1 |
| Condea Augusta S.P.A. | Cosmacol ELI | $C_{12-13}$ Alkyl Lactate | 8,8 |
| ALZO (ROVI) | Dermol 489 | Diethylen Glycol Dioctanoate(/ Diisononanoate | 8,6 |

(fortgesetzt)

| Hersteller | Handelsname | INCI-Name | Polarität mN/m |
|---|---|---|---|
| Condea Augusta S.P.A. | Cosmacol ETI | Di-$C_{12/13}$ Alkyl Tartrate | 7,1 |
| Henkel Cognis | Emerest 2384 | Propylene Glycol Monoisostearate | 6,2 |
| Henkel Cognis | Myritol 331 | Cocoglycerides | 5,1 |
| Unichema | Prisorine 2041 GTIS | Triisostearin | 2,4 |

Besonders vorteilhafte mittelpolare Lipide im Sinne der vorliegenden Erfindung sind die im folgenden aufgelisteten.

| Hersteller | Handelsname | INCI-Name | Polarität (Wasser) mN/m |
|---|---|---|---|
| Henkel Cognis | Cetiol OE | Dicaprylyl Ether | 30,9 |
| | Dihexylcarbonat | Dihexyl Carbonate | 30,9 |
| Albemarle S.A. | Silkflo 366 NF | Polydecene | 30,1 |
| Stearinerie Dubois Fils | DUB VCI 10 | Isodecyl Neopentanoate | 29,9 |
| ALZO (ROVI) | Dermol IHD | Isohexyldecanoate | 29,7 |
| ALZO (ROVI) | Dermol 108 | Isodecyl Octanoate | 29,6 |
| | Dihexyl Ether | Dihexyl Ether | 29,2 |
| ALZO (ROVI) | Dermol 109 | Isodecyl 3,5,5 Trimethyl Hexanoate | 29,1 |
| Henkel Cognis | Cetiol SN | Cetearyl Isononanoate | 28,6 |
| Unichema | Isopropylpalmitat | Isopropylpalmitat | 28,8 |
| Dow Corning | DC Fluid 345 | Cyclomethicone | 28,5 |
| Dow Corning | Dow Corning Fluid 244 | Cyclopolydimethylsiloxan | 28,5 |
| Nikko Chemicals Superior Jojoba Oil Gold | Jojobaöl Gold | | 26,2 |
| Wacker | Wacker AK 100 | Dimethicone | 26,9 |
| ALZO (ROVI). | Dermol 98 | 2- Ethylhexanosäure 3,5,5 Trimethy-. lester | 26,2 |
| Dow Corning | Dow Corning Fluid 246 | Offen | 25,3 |
| Henkel Cognis | Eutanol G | Octyldodecanol | 24,8 |
| Condea Chemie | Isofol 16 | Hexyl Decanol | 24,3 |
| ALZO (ROVI) | Dermol 139 | Isotridecyl 3,5,5 Trimethylhexanonanoate | 24,5 |
| Henkel Cognis | Cetiol PGL | Hexyldecanol (+) Hexyl Decyl Laurate | 24,3 |
| | Cegesoft C24 | Octyl Palmitate | 23,1 |
| Gattefossé | M.O.D. | Octyldodeceyl Myristate | 22,1 |
| | Macadamia Nut Oil | | 22,1 |
| Bayer AG, Dow Corning | Silikonöl VP 1120 | Phenyl Trimethicone | 22,7 |
| CONDEA Chemie | Isocarb 12 | Butyl Octanoicacid | 22,1 |
| Henkel Cognis | Isopropylstearat | Isopropyl Stearate | 21,9 |

(fortgesetzt)

| Hersteller | Handelsname | INCI-Name | Polarität (Wasser) mN/m |
|---|---|---|---|
| WITCO, Goldschmidt | Finsolv TN | C12-15 Alkyl Benzoate | 21,8 |
| Dr. Straetmans | Dermofeel BGC | Butylene Glycol Caprylate/ Caprate | 21,5 |
| Unichema Huels | Miglyol 812 | Caprylic/Capric Triglyceride | 21,3 |
| Trivent (über S. Black) | Trivent OCG | Tricaprylin | 20,2 |
| ALZO (ROVI) | Dermol 866 | PEG "Diethylhexanoate/ Diisononanoate/ Ethylhexyl Isononanoate | 20,1 |

Das oder die anorganischen Verdickungsmittel können beispielsweise vorteilhaft gewählt werden aus der Gruppe der modifizierten oder unmodifizierten, natürlich vorkommender oder synthetischer Schichtsilikate.

Es ist zwar durchaus günstig, reine Komponenten einzusetzen, es können jedoch auch in vorteilhafter Weise, Gemische verschiedener modifizierter und/oder unmodifizierter Schichtsilicate den erfindungsgemäßen Zusammensetzungen einzuverleiben.

Unter Schichtsilicaten, welche auch Phyllosilicate genannt werden, sind im Rahmen dieser Anmeldung Silicate und Alumosilicate zu verstehen, in welchen die Silicat- bzw. Aluminateinheiten über drei Si-O- oder Al-O- Bindungen untereinander verknüpft sind und eine gewellte Blatt- oder Schichtenstruktur ausbilden. Die vierte Si-O- bzw. Al-O-Valenz wird durch Kationen abgesättigt. Zwischen den einzelnen Schichten bestehen schwächere elektrostatische Wechselwirkungen, z.B. Wasserstoffbrückenbindungen. Das Schichtgefüge indessen ist weitgehend durch starke, kovalente Bindungen geprägt.

Die Stöchiometrie der Blattsilicate ist
($Si_2O_5^{2-}$) für reine Silicatstrukturen und
($Al_mSi^{2-}{}_mO_5(^{2+m})^-$) für Alumosilicate.
m ist eine Zahl größer als Null und kleiner als 2.

Liegen keine reinen Silicate sondern Alumosilicate vor, ist dem Umstande Rechnung zu tragen, daß jede durch $Al^{3+}$ ersetzte $Si^{4+}$ - Gruppe ein weiteres einfach geladenes Kation zur Ladungsneutralisierung erfordert.

Die Ladungsbilanz wird bevorzugt durch $H^+$, Alkali- oder Erdalkalimetallionen ausgeglichen. Auch Aluminium als Gegenion ist bekannt und vorteilhaft. Im Gegensatz zu den Alumosilicaten werden diese Verbindungen Aluminiumsilicate genannt. Auch "Aluminiumalumosilicate", in welchen Aluminium sowohl im Silicatnetz, als auch als Gegenion vorliegt, sind bekannt und für die vorliegende Erfindung gegebenenfalls von Vorteil.

Schichtsilicate sind in der Literatur gut dokumentiert, z.B. im "Lehrbuch der Anorganischen Chemie", A.F. Hollemann, E. Wiberg und N. Wiberg, 91.-100. Aufl., Walter de Gruyter - Verlag 1985, passim, sowie "Lehrbuch der Anorganischen Chemie", H.Remy, 12. Aufl., Akademische Verlagsgesellschaft, Leipzig 1965, passim. Die Schichtenstruktur von Montmorillonit ist Römpps Chemie-Lexikon, Franckh'sche Verlagshandlung W. Keller & Co., Stuttgart, 8.Aufl., 1985, S. 2668 f., zu entnehmen.

Beispiele für Schichtsilicate sind:

| | |
|---|---|
| Montmorillonit | $Na_{0,33}((Al_{1,67}Mg_{0,33})(OH)_2(S_{i4}O_{10}))$ |
| oft vereinfacht: | $Al_2O_3{*}4SiO_2{*}H_2O{*}nH_2O$ bzw. $Al_2[(OH)_2/Si_4O_{10}] \cdot n\,H_2O$ |
| Kaolinit | $Al_2(OH)_4(Si_2O_5)$ |
| Ilit | $(K,H_3O)_y(Mg_3(OH)_2(Si_{4-y}Al_yO_{10}))$ |
| und | $(K,H_3O)_y(Al_2(OH)_2(Si_{4-y}Al_yO_{10}))$ mit y = 0,7 - 0,9 |
| Beidellit | $(Ca,Na)_{0,3}(Al_2(OH)_2(Al_{0,5}Si_{3,5}O_{10}))$ |
| Nontronit | $Na_{0,33}(Fe_2(OH)_2(Al_{0,33}Si_{3,67}O_{10}))$ |
| Saponit | $(Ca,Na)_{0,33}((Mg,Fe)_3(OH)_2(Al_{0,33}Si_{3,67}O_{10}))$ |
| Hectorit | $Na_{0,33}((Mg,Li)_3(OH,F)_2(Si_4O_{10}))$ |

Montmorillonit stellt das Hauptmineral der natürlich vorkommenden Bentonite dar.

Sehr vorteilhafte anorganische Gelbildner im Sinne der vorliegenden Erfindung sind Aluminiumsilikate wie die Montmorillonite (Bentonite, Hectorite sowie deren Derivate wie Quaternium-18 Bentonit, Quaternium-18 Hectorite, Stearalkonium Bentonite bzw. Stearalkonium Hectorite) oder aber Magnesium-Aluminium-Silikate (Veegum®-Typen) sowie Natrium-Magnesium-Silikate (Laponite®-Typen)

Montmorillonite stellen zu den dioktaedrischen Smektiten gehörende Tonmineralien dar und sind in Wasser quellende, aber nicht plastisch werdende Massen. Die Schichtpakete in der Dreischicht-Struktur der Montmorillonite können durch reversible Einlagerung von Wasser (in der 2-7fachen Menge) u. a. Substanzen wie z. B. Alkoholen, Glykolen, Pyridin, $\alpha$-Picolin, Ammonium-Verbindungen, Hydroxy-Aluminosilicat-Ionen usw. aufquellen.

Die oben angegebene chemische Formel ist nur angenähert; da M. ein großes Ionenaustausch-Vermögen besitzt, kann Al gegen Mg, $Fe^{2+}$, $Fe^{3+}$, Zn, Pb (z. B. aus Schadstoffen in Abwässern) Cr, auch Cu und andere ausgetauscht werden. Die daraus resultierende negative Ladung der Oktaeder-Schichten wird durch Kationen, insbesondere $Na^+$ (Natrium-Montmorillonit) und $Ca^{2+}$ (der Calcium-Montmorillonit ist nur sehr wenig quellfähig) in Zwischenschicht-Positionen ausgeglichen.

Im Sinne der vorliegenden Erfindung vorteilhafte synthetische Magnesiumsilikate bzw. Bentonite werden beispielsweise von Süd-Chemie unter der Handelsbezeichung Optigel® vertrieben.

Ein im Sinne der vorliegenden Erfindung vorteilhaftes Aluminiumsilikat wird beispielsweise von der R. T. Vanderbilt Comp., Inc., unter der Handelsbezeichnung Veegum® vertrieben. Die verschiedenen Veegum®-Typen, welche alle erfindungsgemäß vorteilhaft sind, zeichnen sich durch folgende Zusammensetzungen aus

| | (regular grade) | HV | K | HS | S-728 |
|---|---|---|---|---|---|
| $SiO_2$ | 55,5 | 56,9 | 64,7 | 69,0 | 65,3 |
| MgO | 13,0 | 13,0 | 5,4 | 2,9 | 3,3 |
| $Al_2O_3$ | 8,9 | 10,3 | 14,8 | 14,7 | 17,0 |
| $Fe_2O_3$ | 1,0 | 0,8 | 1,5 | 1,8 | 0,7 |
| CaO | 2,0 | 2,0 | 1,1 | 1,3 | 1,3 |
| $Na_2O$ | 2,1 | 2,8 | 2,2 | 2,2 | 3,8 |
| $K_2O$ | 1,3 | 1,3 | 1,9 | 0,4 | 0,2 |
| Veraschungsverlust | 11,1 | 12,6 | 7,6 | 5,5 | 7,5 |

Diese Produkte quellen in Wasser unter Bildung viskoser Gele, welche alkalisch reagieren. Durch Organophilierung von Montmorillonit bzw. Bentoniten (Austausch der Zwischenschicht-Kationen gegen quaternäre Alkylammonium-Ionen) entstehen Produkte (Bentone), die bevorzugt zur Dispergierung in organischen Lösemitteln und Ölen, Fetten, Salben, Farben, Lacken und in Waschmitteln eingesetzt werden.

Bentone® ist eine Handelsbezeichnung für verschiedene neutrale und chemisch inerte Geliermittel, die aus langkettigen, organischen Ammoniumsalzen und speziellen Montmorillonit-Sorten aufgebaut sind. Bentone quellen in organischen Medien und bringen diese zum Quellen. Die Gele sind in verdünnten Säuren und Alkalien beständig, bei längerer Berührung mit starken Säuren und Alkalien verlieren sie ihre Geliereigenschaften jedoch teilweise. Aufgrund ihres organophilen Charakters sind die Bentone nur schwer durch Wasser benetzbar.

Folgende Bentone® -Typen werden beispielsweise von der Gesellschaft Kronos Titan vertrieben: Bentone® 27, ein organisch modifiziertes Montmorillonit, Bentone® 34 (Dimethyldioctylammoniumbentonit), das nach US 2,531,427 hergestellt wird und wegen seiner lipophilen Gruppen besser im lipophilen Medium als in Wasser quillt, Bentone® 38, ein organisch modifiziertes Montmorillonit, ein cremefarbenes bis weißes Pulver, Bentone® LT, ein gereinigtes Tonmineral, Bentone® Gel MIO, ein organisch modifiziertes Montmorillonit, das in Mineralöl (SUS-71) feinst suspendiert angeboten wird (10 % Bentonit, 86,7 % Mineralöl und 3,3 % Netzmittel), Bentone® Gel IPM, ein organisch modifiziertes Bentonit, das in Isopropylmyristat suspendiert ist (10 % Bentonit, 86,7 % Isopropylmyristat, 3,3 % Netzmittel), Bentone® Gel CAO, ein organisch modifiziertes Montmorillonit, das in Ricinusöl aufgenommen ist (10 % Bentonit, 86,7 % Ricinusöl und 3,3 % Netzmittel), Bentone® Gel Lantrol, ein organisch modifiziertes Montmorillonit, das in Pastenform zur Weiterverarbeitung, insbesondere zur Herstellung kosmetischer Mittel bestimmt ist; 10 % Bentonit, 64,9 Lantrol (Wollwachsöl), 22,0 Isopropylmyristat, 3,0 Netzmittel und 0,1 p-Hydroxybenzoesäurepropylester, Bentone® Gel Lan I, eine 10 %ige Bentone® 27-Paste in einer Mischung aus Wollwachs USP und Isopropylpalmitat, Bentone® Gel Lan II, eine Bentonit-Paste in reinem, flüssigem Wollwachs, Bentone® Gel NV, eine 15 %ige Bentone® 27-Paste in Dibutylphthalat, Bentone® Gel OMS, eine Bentonit-Paste in Shellsol T. Bentone® Gel OMS 25, eine Bentonit Paste in Isoparaffinischen Kohlenwasserstoffen (Idopar® H), Bentone® Gel IPP, eine Bentonit-Paste in Isopropylpalmitat.

"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die

Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wäßrigen Medien ist. Voraussetzung dafür ist, daß diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

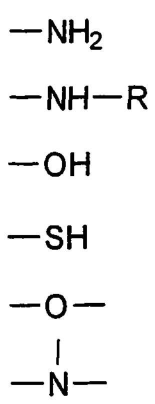
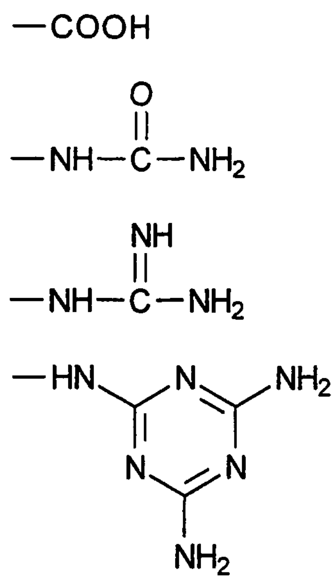
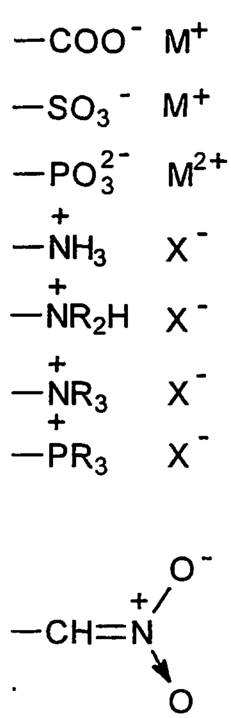
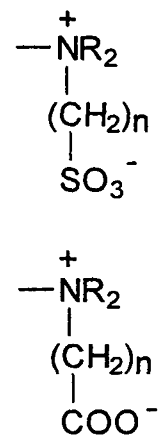

Die Gruppe der kosmetisch und dermatologisch relevanten Hydrokolloide läßt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und mikrokristalline Cellulose dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide, Polyurethane
- anorganische Verbindungen, wie z. B. Polykieselsäuren, Tonmineralien wie Montmorillonite, Zeolithe, Kieselsäuren.

Mikrokristalline Cellulose ist ein vorteilhaftes Hydrokolloid im Sinne der vorliegenden Erfindung. Sie ist beispielsweise von der "FMC Corporation Food and Pharmaceutical Products" unter der Handelsbezeichnung Avicel® erhältlich. Ein besonders vorteilhaftes Produkt im Sinne der vorliegenden Erfindung ist der Typ Avicel® RC-591, bei dem es sich um modifizierte mikrokristalline Cellulose handelt, die sich zu 89% aus mikrokristalliner Cellulose und zu 11 % aus Natrium Carboxymethyl Cellulose zusammensetzt. Weitetere Handelsprodukte dieser Rohstoffklasse sind Avicel® RC/ CL, Avicel® CE-15, Avicel® 500.

Weitere erfindungsgemäß vorteilhafte Hydrokolloide sind beispielsweise Methylcellulosen, als welche die Methylether der Cellulose bezeichnet werden. Sie zeichnen sich durch die folgende Strukturformel aus -

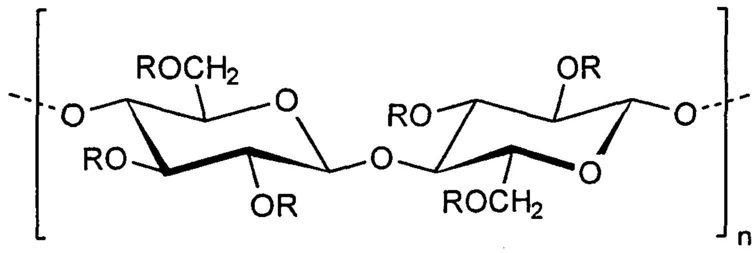

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel® E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder $CH_2$-COONa darstellen kann. Besonders bevorzugt sind die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von $2\times10^6$ bis $24\times10^6$ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 $10^6$ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlantischen, zu den Florideen zählenden Rotalgen (Chondrus crispus und Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht lösl. ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt. Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute ι-Carrageenan ist sowohl wasserlöslich als auch gelbildend. Weitere Carrageen-Typen werden ebenfalls mit griechischen Buchstaben bezeichnet: α, β, γ, μ, ν, ξ, π, ω, χ. Auch die Art vorhandener Kationen ($K^+$, $NH_4^+$, $Na^+$, $Mg^{2+}$, $Ca^{2+}$) beeinflußt die Löslichkeit der Carrageene.

Die Verwendung von Chitosan in kosmetischen Zubereitungen ist per se bekannt. Chitosan stellt ein partiell deacyliertes Chitin dar. Dieses Biopolymer hat u.a. filmbildende Eigenschaften und zeichnet sich durch ein seidiges Hautgefühl aus. Von Nachteil ist jedoch seine starke Klebrigkeit auf der Haut, die insbesondere - vorübergehend - während der Anwendung auftritt. Entsprechende Zubereitungen können dann im Einzelfalle nicht vermarktungsfähig sein, da sie vom Verbraucher nicht akzeptiert bzw. negativ beurteilt werden. Chitosan wird bekanntermaßen beispielsweise in der Haarpflege eingesetzt. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Chitosan ist gekennzeichnet durch folgende Strukturformel:

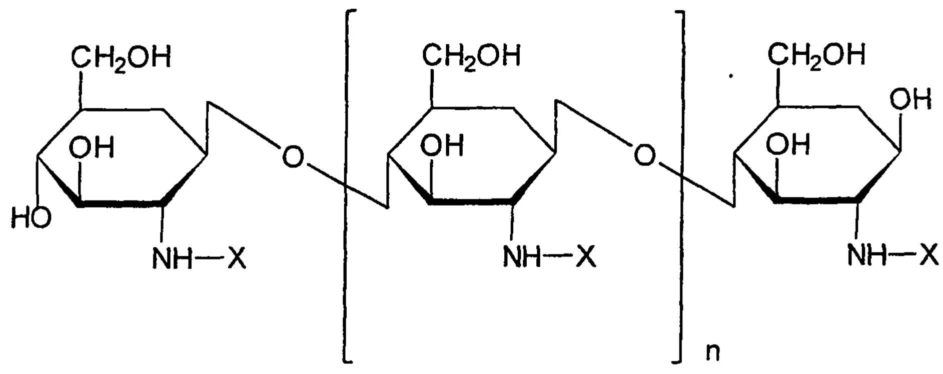

dabei nimmt n Werte bis zu ca. 10.000 an, X stellt entweder den Acetylrest oder Wasserstoff dar. Chitosan entsteht durch Deacetylierung und teilweise Depolymerisation (Hydrolyse) von Chitin, welches durch die Strukturformel

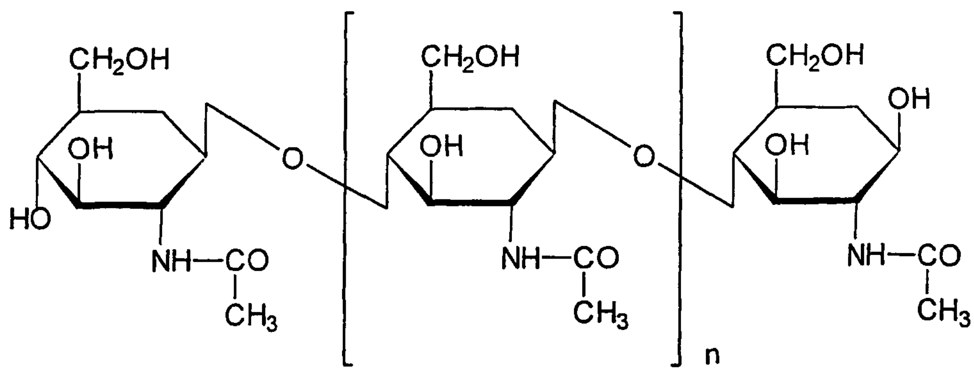

gekennzeichnet ist. Chitin ist wesentlicher Bestandteil des Ektoskeletts ['οχιτων = grch.: der Panzerrock] der Gliederfüßer (z.B. Insekten, Krebse, Spinnen) und wird auch in Stützgeweben anderer Organismen (z.B. Weichtiere, Algen, Pilze) gefunden.

Im Bereich von etwa pH <6 ist Chitosan positiv geladen und dort auch in wäßrigen Systemen löslich. Es ist nicht kompatibel mit anionischen Rohstoffen. Daher bietet sich zur Herstellung chitosanhaltiger Öl-in-Wasser-Emulsionen der Einsatz nichtionischer Emulgatoren an. Diese sind an sich bekannt, beispielsweise aus der EP-A 776 657.

Erfindungsgemäß bevorzugt sind Chitosane mit einem Deacetylierungsgrad > 25 % , insbesondere > 55 bis 99 % [bestimmt mittels $^1$H-NMR]).

Es ist von Vorteil, Chitosane mit Molekulargewichten zwischen 10.000 und 1.000.000 zu wählen, insbesondere solches mit Molekulargewichten zwischen 100.000 und 1.000.000. [bestimmt mittels Gelpermetionschromatographie].

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

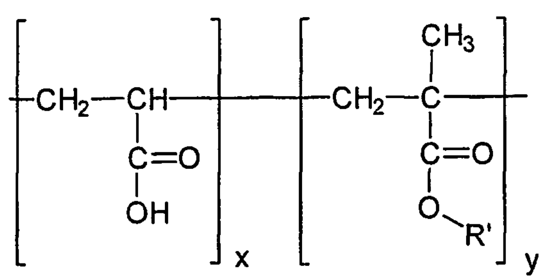

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, bevorzugt Polyacrylate aus der Gruppe der Carbopole der Typen 980, 981, 1382, 2984, 5984 sowie besonders bevorzugt Carbomer 2001

Ferner vorteilhaft sind Copolymere aus $C_{10-30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/$C_{10-30}$ Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Vorteilhaft sind Verbindungen, die die INCI-Bezeichnung Ammoniumacryloyldimethyltaurate/Vinylpyrrolidoncopolymere tragen.

Erfindungsgemäß vorteilhaft weisen das oder die Ammoniumacryloyldimethyltaurate/Vinylpyrrolidoncopolymere die Summenformel $[C_7H_{16}N_2SO_4]_n$ $[C_6H_9NO]_m$ auf, einer statistischen Struktur wie folgt entsprechend .

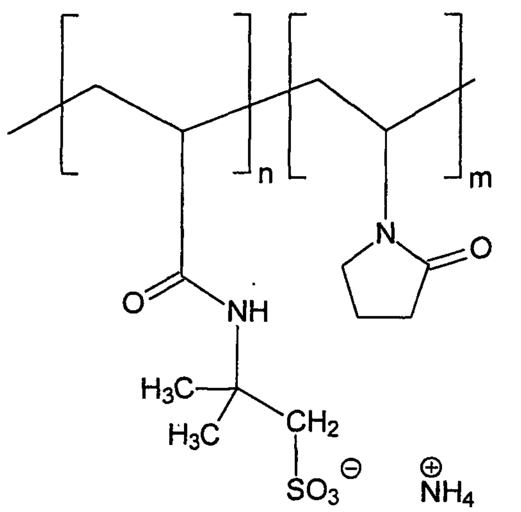

Bevorzugte Spezies im Sinne der vorliegenden Erfindung sind in den Chemical Abstracts unter den Registratumummern 58374-69-9, 13162-05-5 und 88-12-0 abgelegt und erhältlich unter der Handelsbezeichnung Aristoflex® AVC der Gesellschaft Clariant GmbH.

Vorteilhaft sind ferner Copolymere/Crosspolymere umfassend Acryloyldimethyl Taurate, wie beispielsweise Simugel ® EG oder Simugel ® EG von der Gesellschaft Seppic S.A.

Weitere erfindungsgemäß vorteilhaft zu verwendende Hydrokolloide sind auch

1. in Wasser lösliche oder dispergierbare anionische Polyurethane, welche vorteilhaft erhältlich sind aus

   i) mindestens einer Verbindung, die zwei oder mehrere aktive Wasserstoffatome pro Moleküle enthält,
   ii) mindestens einem Säure- oder Salzgruppen enthaltenden Diol und
   iii) mindestens einem Diisocyanat.

Bei der Komponente i) handelt es sich insbesondere um Diole, Aminoalkohole, Diamine, Polyesterole, Polyetherole mit einem zahlenmittleren Molekulargewicht von jeweils bis zu 3000 oder deren Mischungen, wobei bis zu 3 Mol-% der genannten Verbindungen durch Triole oder Triamine ersetzt sein können. Bevorzugt sind Diole und Polyesterdiole. Insbesondere umfaßt die Komponente (a) mindestens 50 Gew.-%, bezogen auf das Gesamtgewicht der Komponente (a), eines Polyesterdiols. Als Polyesterdiole kommen alle diejenigen in Betracht, die üblicherweise zur Herstellung von Polyurethanen eingesetzt werden, insbesondere Umsetzungsprodukte aus Phthalsäure und Diethylenglycol, 1-sophthalsäure und 1,4-Butandiol, Isophthalsäure/Adipinsäure und 1,6-Hexandiol sowie Adipinsäure und Ethylenglycol oder 5-$NaSO_3$-Isophthalsäure, Phthalsäure, Adipinsäure und 1,6-Hexandiol.

Brauchbare Diole sind z.B. Ethylenglycol, Propylenglycol, Butylenglycol, Neopentylglycol, Polyetherole, wie Polyethylenglycole mit Molekulargewichten bis zu 3000, Blockcopolymerisate aus Ethylenoxid und Propylenoxid mit

zahlenmittleren Molekulargewichten von bis zu 3000 oder Blockcopolymerisate aus Ethylenoxid, Propylenoxid und Butylenoxid, die die Alkylenoxideinheiten statistisch verteilt oder in Form von Blöcken einpolymerisiert enthalten. Bevorzugt sind Ethylenglycol, Neopentylglycol, Di-, Tri-, Tetra-, Penta oder Hexaethylenglyol. Brauchbare Diole sind außerdem Poly(a-hydroxycarbonsäure)diole.

Geeignete Aminoalkohole sind z.B. 2-Aminoethanol, 2-(N-Methylamino)ethanol, 3-Aminopropanol oder 4-Aminobutanol.

Geeignete Diamine sind z.B. Ethylendiamin, Propylendiamin, 1,4-Diaminobutan und 1,6-Diaminohexan sowie $\alpha,\omega$-Diamine, die durch Aminierung von Polyalkylenoxiden mit Ammoniak herstellbar sind.

Bei der Komponente ii) handelt es sich insbesondere um Dimethylolpropansäure oder Verbindungen der Formeln

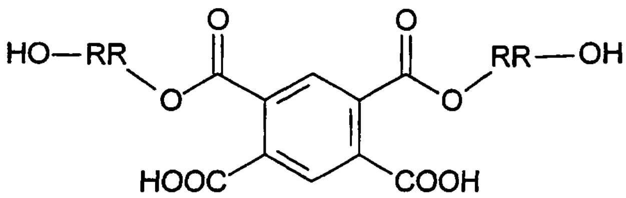

bzw.

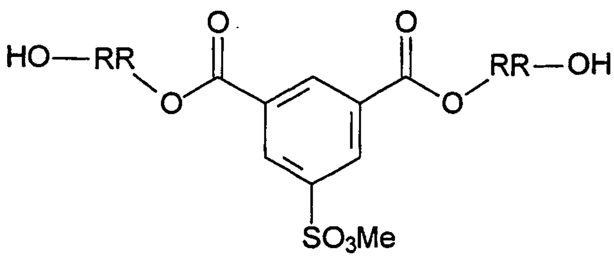

worin RR jeweils für eine $C_2$-$C_{18}$-Alkylengruppe steht und Me für Na oder K steht.

Bei der Komponente iii) handelt es sich insbesondere um Hexamethylendiisocyanat, Isophorondiisocyanat, Methyldiphenylisocyanat (MDI) und/oder Toluylendiisocyanat.

Die Polyurethane sind dadurch erhältlich, daß man die Verbindungen der Gruppen i) und ii) unter einer Inertgasatmosphäre in einem inerten Lösemittel bei Temperaturen von 70 bis 130˚C mit den Verbindungen der Gruppe iii) umsetzt. Diese Umsetzung kann gegebenenfalls in Gegenwarte von Kettenverlängerern durchgeführt werden, um Polyurethane mit höheren Molekulargewichten herzustellen. Wie bei der Herstellung Polyurethanen üblich, werden die Komponenten [(i)+(ii)]:iii) vorteilhaft im molaren Verhältnis von 0,8 bis 1,1 : 1 eingesetzt. Die Säurezahl der Polyurethane wird von der Zusammensetzung und der Konzentration der Verbindungen der Komponente (ii) in der Mischung aus den Komponenten (i)+(ii) bestimmt.

Die Polyurethane haben K-Werte nach H. Fikentscher (bestimmt in 0,1 gew.-%igen Lösungen in N-Methylpyrrolidon bei 25 ˚C und pH 7) von 15 bis 100, vorzugsweise 25 bis 50.

Der auch als Eigenviskosität bezeichnete K-Wert ist ein über Viskositätsmessungen von Polymerlösungen einfach zu bestimmender und daher im techn. Bereich häufig benützter Parameter zur Charakterisierung von Polymeren. Für eine bestimmte Polymer-Sorte wird er unter standardisierten Meßbedingungen als alleine abhängig von der mittleren Molmasse der untersuchten Probe angenommen und über die Beziehung K-Wert = 1000 k nach der Fikentscher-Gleichung

$$k = \frac{1{,}5\lg\eta_r - 1 \pm \sqrt{1 + \left(\frac{2}{c} + 2 + 1{,}5\lg\eta_r\right)\cdot 1{,}5\lg\eta_r}}{150 + 300c}$$

berechnet, in der bedeuten: $\eta_r$ = relative Viskosität (dynamische Viskosität der Lösung/dynamische Viskosität des Lösemittels) und c = Massenkonzentration an Polymer in der Lösung (in $g/cm^3$).

Die Säuregruppen enthaltenden Polyurethane sind nach Neutralisation (teilweise oder vollständig) wasserlöslich bzw. ohne Zuhilfenahme von Emulgatoren dispergierbar. In aller Regel weisen die Salze der Polyurethane eine bessere Wasserlöslichkeit oder Dispergierbarkeit in Wasser auf als die nicht neuralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Ammoniak und Amine verwendet werden. Besonders haben sich zur Neutralisation der Säuregruppen enthaltenden Polyurethane 2-Amino-2-Methylpropanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z.B. Mischungen aus Natronlauge und Triisopropanolamin. Die Neutralisation kann je nach Anwendungszweck partiell z.B. zu 20 bis 40 % oder vollständig, d.h. zu 100 % erfolgen.

Diese Polymere und ihre Herstellung sind in DE-A-42 25 045 näher beschrieben, auf die hiermit in vollem Umfang Bezug genommen wird.

2. In Wasser lösliche oder dispergierbare, kationische Polyurethane und Polyharnstoffe aus

a) mindestens einem Diisocyanat, welches bereits vorher mit einer oder mehreren Verbindungen, die zwei oder mehrere aktive Wasserstoffatome pro Moleküle enthalten, umgesetzt worden sein kann, und
b) mindestens einem Diol, primären oder sekundären Aminoalkohol, primären oder sekundärem Diamin oder primären oder sekundären Triamin mit einem oder mehreren tertiären, -quaternären oder protonierten tertiären Aminostickstoffatomen.

Bevorzugte Diisocyanate sind wie oben unter 1) angegeben. Verbindungen mit zwei oder mehreren aktiven Wasserstoffatomen sind Diole, Aminoalkohole, Diamine, Polyesterole. Polyamiddiamine und Polyetherole. Geeignete Verbindungen dieser Art sind wie oben unter 1) angegeben.

Die Herstellung der Polyurethane erfolgt wie oben unter 1) beschrieben. Geladene kationische Gruppierungen lassen sich aus den vorliegenden tertiären Aminostickstoffatomen entweder durch Protonierung, z.B. mit Carbonsäuren wir Milchsäure, oder durch Quaternisierung, z.B. mit Alkylierungsmitteln wie $C_1$-bis $C_4$-Alkylhalogeniden oder -sulfaten in den Polyharnstoffen erzeugen. Beispiele solcher Alkylierungsmittel sind Ethylchlorid, E-thylbromid, Methylchlorid, Methylbromid, Dimethylsulfat und Diethylsulfat.

Diese Polymere und ihre Herstellung sind in der DE-A-42 41 118 näher beschrieben, auf die hiermit in vollem Umfang Bezug genommen wird.

3. Lineare Polyurethane mit Carboxylatgruppen aus

i) einer 2,2-Hydroxymethyl-substituierten Carbonsäure der Formel

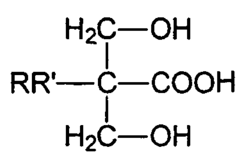

worin RR' für ein Wasserstoffatom oder eine $C_1$-$C_{20}$-Alkylgruppe steht, die in einer Menge verwendet wird, welche ausreicht, daß in dem Polyurethan 0,35 bis 2,25 Milliäquivalente Carboxylgruppen pro g Polyurethan vorhanden sind,

ii) 10 bis 90 Gew.-%, bezogen auf das Gewicht des Polyurethans, einer oder mehrerer organischer Verbindungen mit nicht mehr als zwei aktiven Wasserstoffatomen und

iii) einem oder mehreren organischen Diisocyanaten.

Die im Polyurethan enthaltenden Carboxylgruppen werden abschließend mit einer geeigneten Base zumindest teilweise neutralisiert. Diese Polymere und ihre Herstellung sind in der EP-A-619 111 beschrieben, auf die hiermit in vollem Umfang Bezug genommen wird.

4. Carboxylhaltige Polykondensationsprodukte aus Anhydriden von Tri- oder Tetracarbonsäuren und Diolen,

Diaminen oder Aminoalkoholen (Polyester, Polyamide oder Polyesteramide). Diese Polymere und ihre Herstellung sind in der DE-A-42 24 761 näher beschrieben, auf die hiermit in vollem Umfang Bezug genommen wird.

5. Polyacrylate und Polymethacrylate, wie sie in den DE-A-43 14 305, 36 27 970 und 29 17 504 näher beschrieben sind. Auf diese Publikationen wird hiermit in vollem Umfang Bezug genommen.

Die erfindungsgemäß zur Anwendung kommenden Polymere besitzen vorzugsweise einen K-Wert von 25 bis 100, bevorzugt 25 bis 50. Die Polymere sind in dem erfindungsgemäßen Mittel im allgemeinen in einer Menge im Bereich von 0,2 bis 20 Gew.-%, bezogen auf das Gesamtgewicht des Mittels, enthalten. Das Salz kommt in einer zur Verbesserung der Austauschbarkeit der Polymeren wirksamen Menge zur Anwendung. Im allgemeinen setzt man das Salz in einer Menge von 0,02 bis 10 Gew.-%, vorzugsweise 0,05 bis 5 Gew.-% und insbesondere 0,1 bis 3 Gew.-%, bezogen auf das Gesamtgewicht des Mittels, ein.

Die Gesamtmenge an einem oder mehreren Hydrokolloiden wird in den fertigen kosmetischen oder dermatologischen Zubereitungen vorteilhaft kleiner als 5% Gew. %, bevorzugt zwischen 0,1 und 1,0 Gew.-%, bezogen auf das Gesamtgewicht der Zubereitungen, gewählt.

Die erfindungsgemäßen kosmetischen und/oder dermatologischen Zubereitungen können wie üblich zusammengesetzt sein. Besonders vorteilhaft im Sinne der vorliegenden Erfindung sind Zubereitungen zur Pflege der Haut: sie können dem kosmetischen und/oder dermatologischen Lichtschutz, ferner zur Behandlung der Haut und/oder der Haare und als Schminkprodukt in der dekorativen Kosmetik dienen. Eine weitere vorteilhafte Ausführungsform der vorliegenden Erfindung besteht in After-Sun-Produkten.

Entsprechend ihrem Aufbau können kosmetische oder topische dermatologische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Ebenso wie Emulsionen von flüssiger und fester Konsistenz als kosmetische Reinigungslotionen bzw. Reinigungscremes Verwendung finden, können auch die erfindungsgemäßen Zubereitungen "Reinigungsschäume" darstellen, welche beispielsweise zum Entfernen von Schminken und/oder Make-up oder als milder Waschschaum - ggf. auch für unreine Haut - verwendet werden können. Derartige Reinigungsschäume können vorteilhaft ferner als sogenannte "rinse off" Präparate angewendet werden, welche nach der Anwendung von der Haut abgespült werden

Die erfindungsgemäßen kosmetischen und/oder dermatologischen Zubereitungen können auch vorteilhaft in Form eines Schaums zur Pflege des Haars bzw. der Kopfhaut vorliegen, insbesondere eines Schaums zum Einlegen der Haare, eines Schaums, der beim Fönen der Haare verwendet wird, eines Frisier- und Behandlungsschaums.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Farbstoffe, Pigmente, die eine färbende Wirkung haben, anfeuchtende und/oder feuchthaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin), lodopropylbutylcarbamate (z. B. die unter den Handelsbezeichnungen Koncyl-L, Koncyl-S und Konkaben LMB von der Fa. Lonza erhältlichen), Parabene, Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D, L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis μmol/kg), ferner (Metall)-Chelatoren (z. B. α-Hydroxyfettsäuren, Palmitinsäure, Phytinsäure, Lactoferrin), α-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg - Ascorbylphosphat, Ascorbylacetat), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO4) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Eine erstaunliche Eigenschaft der erfindungsgemäße Zubereitungen ist, daß diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z. B. Hydrocortison-17-valerat, Vitamine der B- und D-Reihe, sehr günstig das Vitamin $B_{1,}$ das Vitamin $B_{12}$ das Vitamin $D_1$, aber auch Bisabolol, ungesättigte Fettsäuren, namentlich die essentiellen Fettsäuren (oft auch Vitamin F genannt), insbesondere die gamma-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

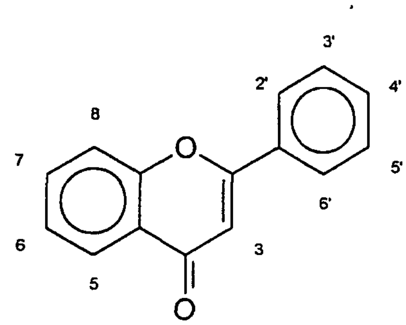

Einige der wichtigeren Flavone, welche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt gewählt aus der Gruppe der Substanzen der generischen Strukturformel

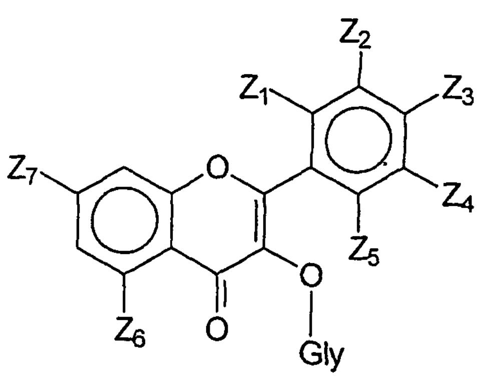

,

wobei $Z_1$ bis $Z_7$ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und

wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel

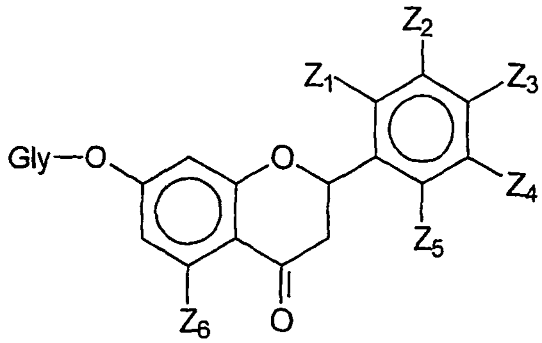

,

wobei $Z_1$ bis $Z_6$ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel

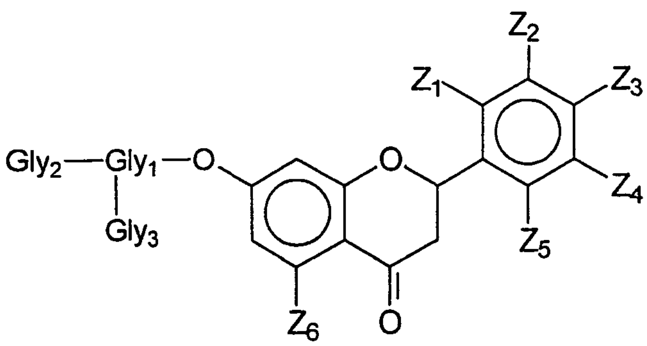

wobei $Gly_1$, $Gly_2$ und $Gly_3$ unabhängig voneinander Monoglycosidreste oder darstellen. $Gly_2$ bzw. $Gly_3$ können auch einzeln oder gemeinsam Absättigungen durch Wasserstoffatome darstellen.

Bevorzugt werden $Gly_1$, $Gly_2$ und $Gly_3$ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden $Z_1$ bis $Z_5$ unabhängig voneinander gewählt aus der Gruppe H, OH, Methoxy-, Ethoxy- sowie 2-Hydroxyethoxy-, und die Flavonglycoside haben die Struktur

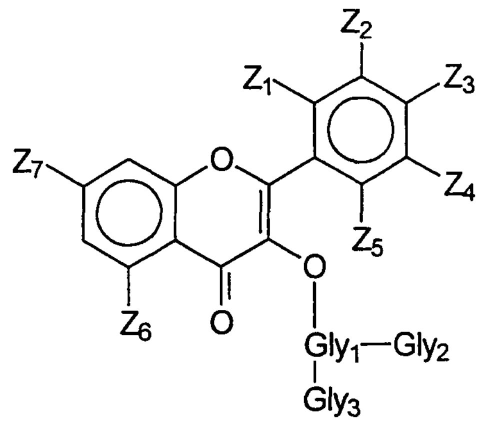

Besonders vorteilhaft werden die erfindungsgemäßen Flavonglycoside aus der Gruppe, welche durch die folgende Struktur wiedergegeben werden:

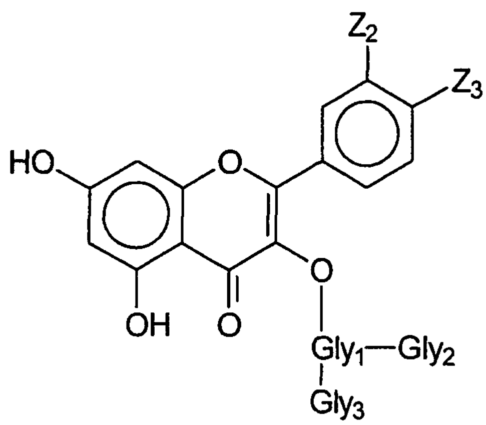

wobei $Gly_1$, $Gly_2$ und $Gly_3$ unabhängig voneinander Monoglycosidreste oder darstellen. $Gly_2$ bzw. $Gly_3$ können auch einzeln oder gemeinsam Absättigungen durch Wasserstoffatome darstellen.

Bevorzugt werden $Gly_1$, $Gly_2$ und $Gly_3$ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy- 3', 4', 7- tris (2- hydroxyethoxy)-flavon- 3-(6- O-(6- deoxy- α- L- mannopyranosyl)- β- D- glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

Ubichinone zeichnen sich durch die Strukturformel

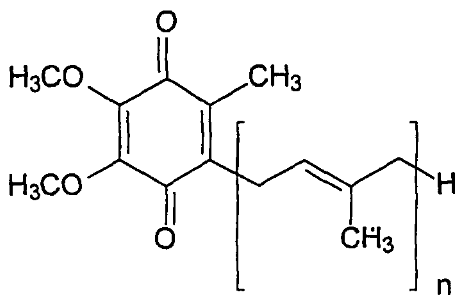

,

aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

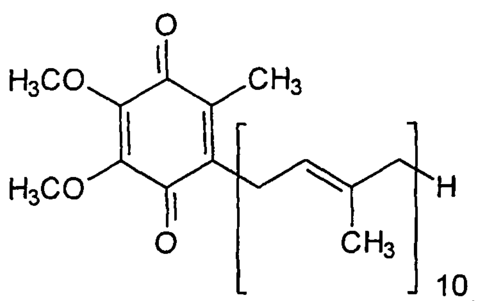

Plastochinone weisen die allgemeine Strukturformel

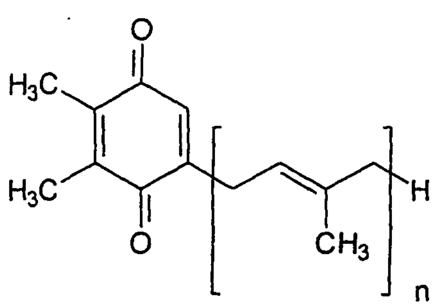

auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus:

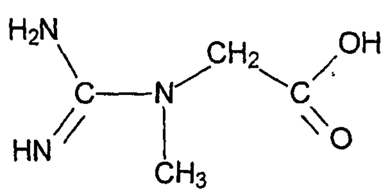

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Camitin [3-Hydroxy-4-(trimethylammonio)-buttersäurebetain]. Auch Acyl-Carnitine, welche gewählt aus der Gruppe der Substanzen der folgenden allgemeinen Strukturformel

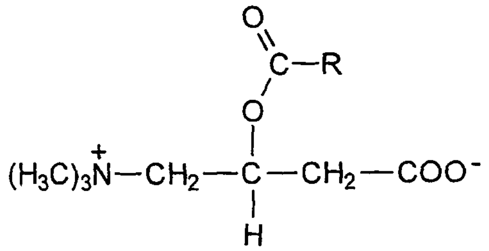

,

wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Vitamin K, Biotin und Aromastoffe.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:

a) Trockenheit, Rauhigkeit und Ausbildung von (Trockenheits-) Fältchen,
b) Juckreiz und
c) verminderte Rückfettung durch Talgdrüsen (z. B. nach dem Waschen).

Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:

d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

Erstaunlicherweise können ausgewählte erfindungsgemäße Rezepturen auch eine Antifaltenwirkung aufweisen bzw. die Wirkung bekannter Antifaltenwirkstoffe erheblich steigern. Dementsprechend eignen sich Formulierungen im Sinne der vorliegenden Erfindung insbesondere vorteilhaft zur Prophylaxe und Behandlung kosmetischer oder dermatologischer Hautveränderungen, wie sie z. B. bei der Hautalterung auftreten. Weiterhin vorteilhaft eignen sie sich gegen das Erscheinungsbild der trockenen bzw. rauhen Haut.

In einer besonderen Ausführungsform betrifft die vorliegende Erfindung daher Produkte zur Pflege der auf natürliche Weise gealterten Haut, sowie zur Behandlung der Folgeschäden der Lichtalterung, insbesondere der unter a) bis g) aufgeführten Phänomene.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der

Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphthalin-7-suifosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure 2-(2,4-Dimethylphenylazo-5-sulfosäure)-1-hydroxynaphthalin-4-sulfosäure | 14270 14700 | orange rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 1.5525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure. | 16185 | rot |
| Acid Orange 10 | 16230 | orange |

(fortgesetzt)

| Chemische oder sonstige Bezeichnung | CIN | Farbe |
|---|---|---|
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd ($C_{30}$) | 40820 | orange |
| trans-Apo-8'-Carotinsäure ($C_{30}$)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophe- | 42053 | grün |
| nyl)-(methylen)-1-(N-ethylN-p-sulfobenzyl)-2,5-cyclohexadienimin] Acid Blue 7 | 42080 | blau |
| (N-Ethyl-p-sulfobenzyl-amino)-phenyl-(2-sulfophenyl)-methylen-(N-ethyl-N-p-sulfo-benzyl)$\Delta^{2,5}$-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |

(fortgesetzt)

| Chemische oder sonstige Bezeichnung | CIN | Farbe |
|---|---|---|
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure . | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |

(fortgesetzt)

| Chemische oder sonstige Bezeichnung | CIN | Farbe |
|---|---|---|
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; $Mn_3(PO_4)_2 \cdot$ 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, . 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

   - "Fischsilber" (Guanin/Hypoxanthin-Mischkristalle aus Fischschuppen) und
   - "Perlmutt" (vermahlene Muschelschalen)

2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | $TiO_2$: 40 - 60 nm | silber |
| Interferenzpigmente | $TiO_2$: 60 - 80 nm | gelb |
| | $TiO_2$: 80-100 nm | rot |
| | $TiO_2$: 100-140 nm | blau |
| | $TiO_2$: 120-160 nm | grün |
| Farbglanzpigmente | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ $Fe_2O_3$ | rotgrün schwarz |
| Kombinationspigmente | $TiO_2$ / $Fe_2O_3$ | Gotdtöne |
| | $TiO_2$ /$Cr_2O_3$ $TiO_2$ / Berliner Blau | grün tiefblau |
| | $TiO_2$ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%. insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise neben einer oder mehreren erfindungsgemäßen UV-Filtersubstanzen zusätzlich mindestens eine weitere UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$),

Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden.

Solche Pigmente können im Sinne der vorliegenden Erfindung vorteilhaft oberflächlich behandelt ("gecoatet") sein, wobei beispielsweise ein amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Erfindungsgemäß vorteilhaft sind z. B. Titandioxidpigmente, die mit Octylsilanol beschichtet sind. Geeignete Titandioxidpartikel sind unter der Handelsbezeichnung T805 bei der Firma Degussa erhältlich. Besonders vorteilhaft sind ferner mit Aluminiumstearat beschichtete $TiO_2$-Pigmente, z. B. die unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel

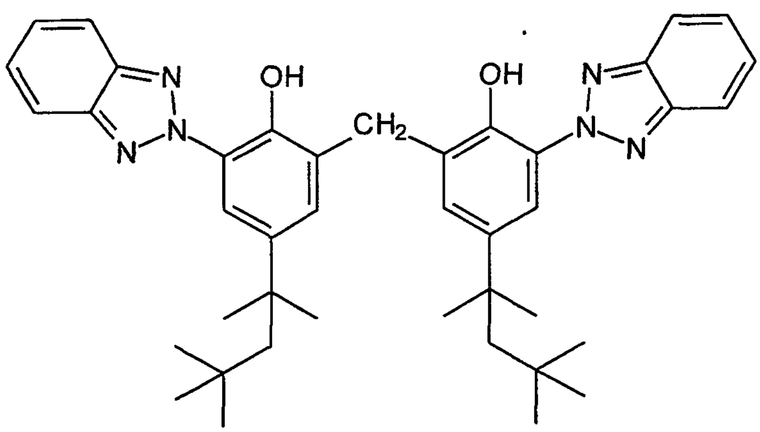

gekennzeichnet ist und unter der Handelsbezeichnung Tinosorb® M bei der CIBA-Chemikalien GmbH erhältlich ist.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure

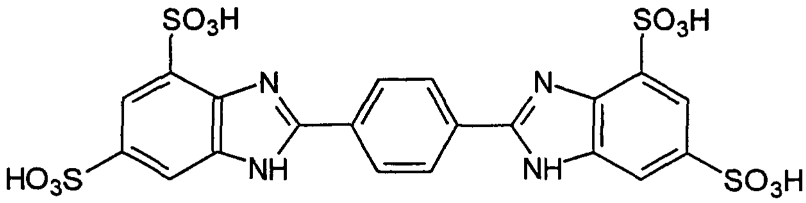

und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz

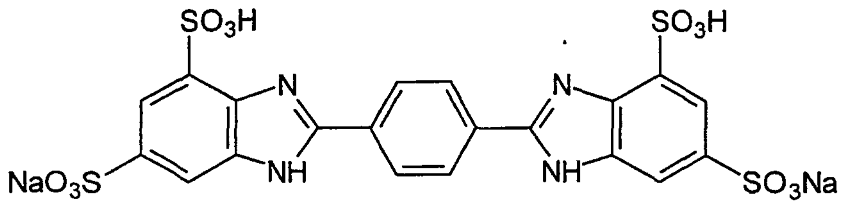

mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

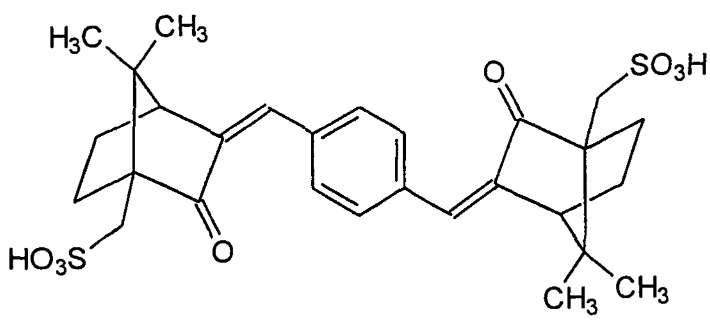

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur:

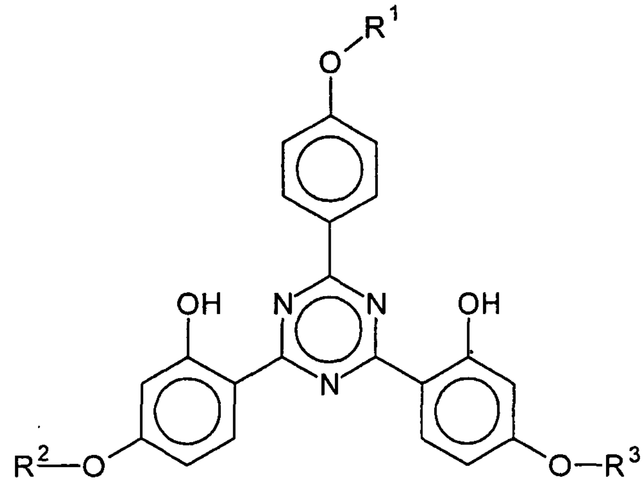

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1.3,5-triazin, jeweils einzeln oder in beliebigen Kombinationen miteinander.

Auch andere UV-Filtersubstanzen, welche das Strukturmotiv

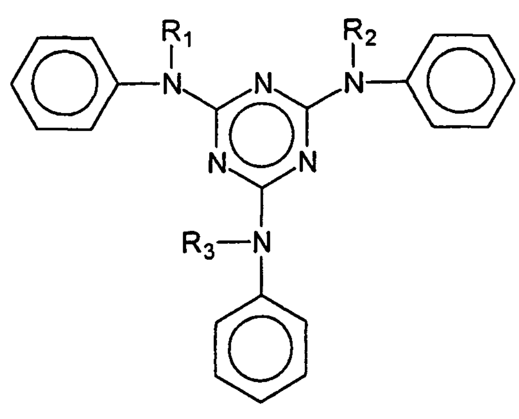

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel

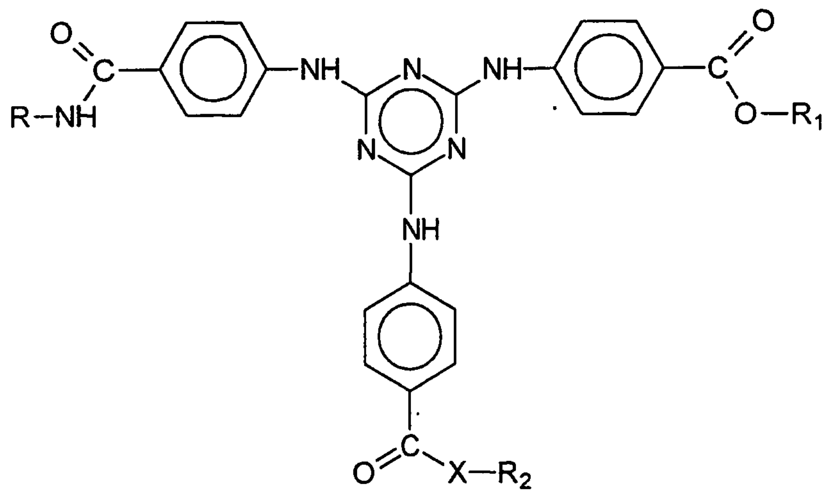

wiedergegeben wird, wobei

R einen verzweigten oder unverzweigten $C_1$-$C_{18}$Alkylrest, einen $C_5$-$C_{12}$-Cycloalkylrest, gegebenenfalls substituiert mit einer oder mehreren $C_1$-$C_4$- Alkylgruppen, darstellt,
X ein Sauerstoffatom oder eine NH-Gruppe darstellt,
$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

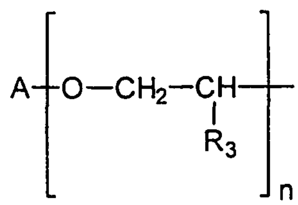

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,
$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und

einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

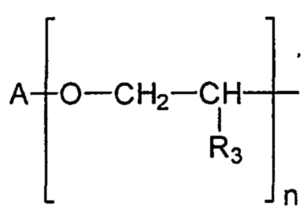

bedeutet, in welcher

- A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
- $R_3$ ein Wasserstoffatom oder eine Methylgruppe darstellt,
- n eine Zahl von 1 bis 10 darstellt,

wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel

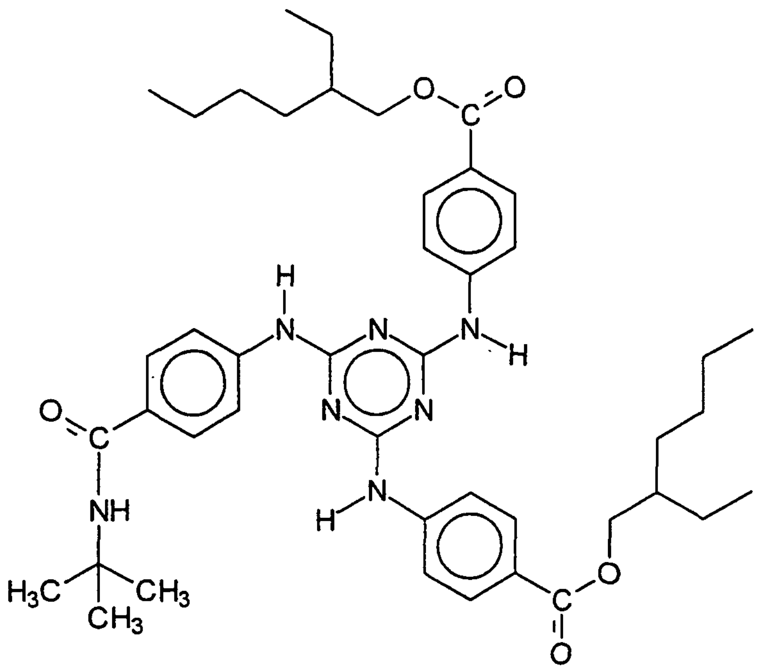

wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4', 4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel

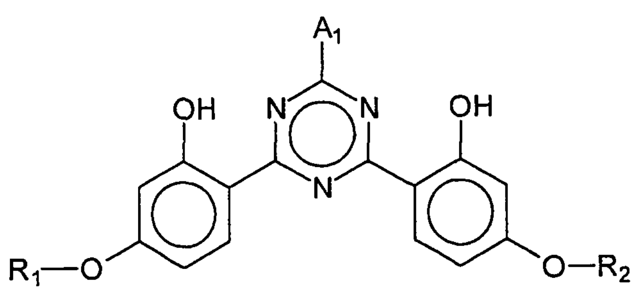

wiedergegeben wird, wobei $R_1$, $R_2$ und $A_1$ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel

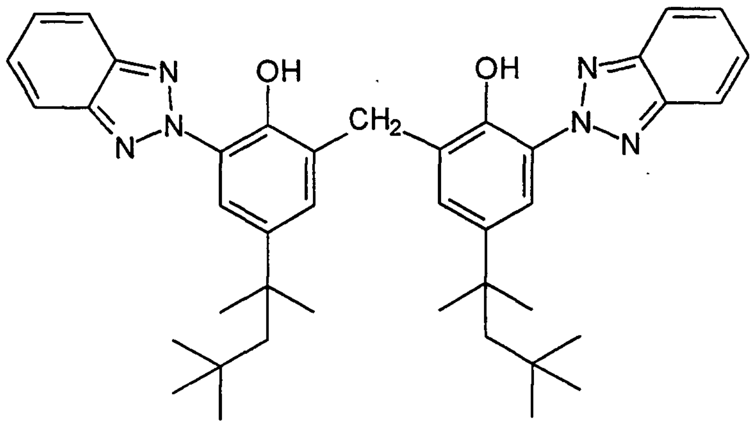

gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel

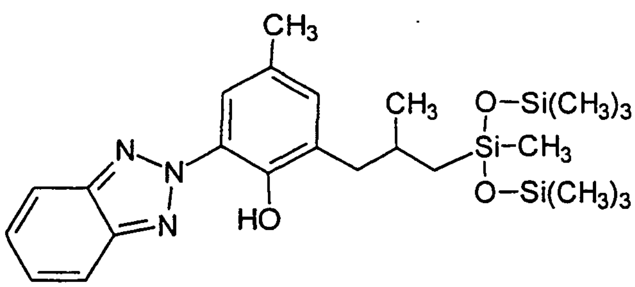

gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Vorteilhafte wasserlösliche UV-B- und/oder Breitband-Filtersubstanzen sind z. B.:

- ■ Salze der 2-Phenylbenzimidazol-5-sulfonsäure, wie ihr Natrium-, Kalium- oder ihr Triethanolammonium-Salz, sowie die Sulfonsäure selbst;
- ■ Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

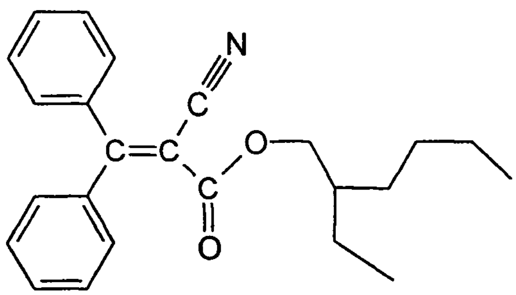

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/ oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiele:**

**Beispiel 1 (schaumförmige O/W-Creme):**

| Emulsion I | Gew.-% | Vol.-% |
|---|---|---|
| Stearinsäure | 3,00 | |
| Cetylalkohol | 8,50 | |
| PEG-20-Stearat | 8,50 | |
| Polyacrylsäure | 0,20 | |
| Magnesiumaluminiumsilikat | 0,50 | |
| $C_{12-15}$Alkylbenzoat | 4,00 | |
| Paraffinöl | 5,00 | |
| Isohexadecan | 2,00 | |
| Glycerin | 5,00 | |
| Natriumhydroxid | q.s. | |
| Konservierung | q.s. | |

(fortgesetzt)

| Emulsion I | Gew.-% | Vol.-% |
|---|---|---|
| Parfum | q.s. | |
| Wasser, demineralisiert | ad 100,00 | |
| pH-Wert eingestellt auf 6,5-7,5 | | |
| Emulsion I | | 70 |
| Stickstoff | | 30 |

Vordispergierung des anorganischen Gelbildners und Quellung des Hydrokolloides unter Rühren in der Wasserphase. Vereinigung der auf 75˚C aufgeheizten Fettphase mit der auf 70˚C aufgeheizten Wasserphase. Homogenisierung mittels einer Zahnkranzdispergiermaschine (Rotor-Stator-Prinzip) bei 65˚C. 45 min Rühren unter Begasung mit Stickstoff bei 0.7bar und Kühlung. Zugabe der Additive bei 30˚C (Parfüm, Wirkstoffe). Homogenisierung mittels einer Zahnkranzdispergiermaschine (Rotor-Stator-Prinzip) bei 27˚C.

**Beispiel 2 (schaumförmige O/W-Lotion):**

| Emulsion II | Gew.-% | Vol.-% |
|---|---|---|
| Stearinsäure | 2,00 | |
| Myristylalcohol | 1,50 | |
| Cetylstearylalcohol | 0,50 | |
| PEG-100 Stearate | 3,00 | |
| Polyacrylsäure | 0,20 | |
| Magnesium-Aluminium-Silikate | 0,20 | |
| Mineralöl | 5,00 | |
| Hydriertes Polyisobuten | 15,0 | |
| Glycerin | 3,00 | |
| Natriumhydroxid | q.s. | |
| Konservierung | q.s. | |
| Parfum | q.s. | |
| Wasser,demineralisiert | ad 100,00 | |
| pH-Wert eingestellt auf 5,0-6,5 | | |
| Emulsion II | | 50 |
| Gas (Kohlendioxid) | | 50 |

Vordispergierung des anorganischen Gelbildners und Quellung des Hydrokolloides unter Rühren in der Wasserphase. Vereinigung der auf 80˚C aufgeheizten Fettphase mit der auf 72˚C aufgeheizten Wasserphase. Homogenisierung mittels einer Zahnkranzdispergiermaschine (Rotor-Stator-Prinzip) bei 65˚C. 45 min Rühren unter Begasung mit Kohlendioxid bei 1.2 bar und Kühlung. Zugabe der Additive bei 30˚C (Parfüm). Homogenisierung mittels einer Zahnkranzdispergiermaschine (Rotor-Stator-Prinzip) bei 30˚C.

**Beispiel 3 (schaumförmige O/W-Lotion):**

| Emulsion III | Gew.-% | Vol.-% |
|---|---|---|
| Stearinsäure | 5,00 | |
| Cetylstearylalkohol | 5,50 | |
| PEG-30-Stearat | 1,00 | |
| Xanthangummi | 0,10 | |
| Cellulosegummi | 0,10 | |
| Magnesiumsilikat | 0,10 | |

(fortgesetzt)

| Emulsion III | Gew.-% | Vol.-% |
|---|---|---|
| Cyclomethicon | 3,00 | |
| Isoeikosan | 10,00 | |
| Polydecen | 10,00 | |
| Citronensäure | 0,10 | |
| Glycerin | 3,00 | |
| Parfüm, Konservierungsmittel, | q.s. | |
| Natriumhydroxid | q.s. | |
| Farbstoffe usw. | q.s. | |
| Wasser | ad 100,00 | |
| pH-Wert eingestellt auf 6,0-7,5 | | |
| Emulsion III | | 65 |
| Gas (Luft) | | 35 |

Vordispergierung des anorganischen Gelbildners und Quellung der Hydrokolloide unter Rühren in der Wasserphase. Vereinigung der auf 80˚C aufgeheizten Fettphase mit der auf 75˚C aufgeheizten Wasserphase. Homogenisierung mittels einer Zahnkranzdispergiermaschine (Rotor-Stator-Prinzip) bei 65˚C. 45 min Rühren in einem offenen Kessel bis auf 30˚C. Zugabe der Additive bei 30˚C (Parfüm, Wirkstoffe). Homogenisierung mittels einer Zahnkranzdispergiermaschine (Rotor-Stator-Prinzip) bei 25˚C.

**Beispiel 4 (schaumförmige O/W-Emulsions-Make-up):**

| Emulsion IV | Gew.-% | Vol.-% |
|---|---|---|
| Palmitinsäure | 2,00 | |
| Cetylalkohol | 2,00 | |
| PEG-100-Stearat | 2,00 | |
| Natriummagnesiumsilicat | 0,50 | |
| Dimethicon | 0,50 | |
| Paraffinöl | 9,50 | |
| Dicaprylylether | 2,00 | |
| Glycerin | 3,00 | |
| Glimmer | 1,00 | |
| Eisenoxide | 1,00 | |
| Titandioxid | 4, 50 | |
| Vitamin-A-Palmitat | 0,10 | |
| Hectorit | 0,10 | |
| Polyacrylsäure | 0,15 | |
| Natriumhydroxid | q.s. | |
| Konservierung | q.s. | |
| Parfum | q.s. | |
| Wasser,demineralisiert | ad 100,00 | |
| pH-Wert eingestellt auf 6,0 -7,5 | | |
| Emulsion IV | | 37 |
| Gas (Sauerstoff) | | 63 |

Vordispergierung der anorganischen Gelbildner und Quellung des Hydrokolloids unter Rühren in der Wasserphase. Vereinigung der auf 78˚C aufgeheizten Fett- und Pigmentphase mit der auf 75˚C aufgeheizten Wasserphase. Homogenisierung mittels einer Zahnkranzdispergiermaschine (Rotor-Stator-Prinzip) bei 65˚C. 45 min Rühren im Becomix unter Begasung mit Sauerstoff bei 1,3 bar unter Kühlung auf 30˚C. Zugabe der Additive bei 30˚C (Parfüm, Wirkstoffe). Homogenisierung mittels einer Zahnkranzdispergiermaschine (Rotor-Stator-Prinzip) bei 25˚C.

**Beispiel 5 (schaumförmige O/W-Creme):**

| Emulsion V | Gew.-% | Vol.-% |
|---|---|---|
| Stearinsäure | 4,00 | |
| Cetylalkohol | 2,00 | |
| PEG-30-Stearat | 2,00 | |
| Sorbitanmonostearat | 1,50 | |
| Paraffinöl | 5,00 | |
| Cyclomethicon | 1,00 | |
| Vitamin-E-Acetat | 1,00 | |
| Retinylpalmitat | 0,20 | |
| Glycerin | 3,00 | |
| BHT | 0,02 | |
| $Na_2H_2EDTA$ | 0,10 | |
| Bentonit | 0,05 | |
| Magnsiumaluminiumsilikate | 0,10 | |
| Polyacrylsäure | 0,35 | |
| Xanthangummi | 0,10 | |
| Carrageen | 0,05 | |
| Parfüm, Konservierungsmittel, | q.s. | |
| Farbstoffe | q.s. | |
| Kaliumhydroxid | q.s. | |
| Wasser | ad 100,00 | |
| pH-Wert eingestellt auf 5,0-7,0 | | |
| Emulsion V | | 43 |
| Gas (Lachgas) | | 57 |

Vordispergierung der anorganischen Gelbildner und Quellung des Hydrokolloids unter Rühren in der Wasserphase. Vereinigung der auf 80˚C aufgeheizten Fettphase mit der auf 75˚C aufgeheizten Wasserphase. Homogenisierung mittels einer Zahnkranzdispergiermaschine (Rotor-Stator-Prinzip) bei 65˚C. 45 min Rühren im Becomix unter Begasung mit Lachgas bei 0.7 bar unter Kühlung auf 30˚C. Zugabe der Additive bei 30˚C (Parfüm, Wirkstoffe). Homogenisierung mittels einer Zahnkranzdispergiermaschine (Rotor-Stator-Prinzip) bei 26˚C.

**Beispiel 6 (schaumförmige O/W-Lotion):**

| Emulsion VI | Gew.-% | Vol.-% |
|---|---|---|
| Stearinsäure | 4,00 | |
| Cetylstearylalkohol | 1,00 | |
| PEG-100-Stearat | 1,00 | |
| Paraffinöl | 6,50 | |
| Dimethicon | 0,50 | |
| Vitamin-E-Acetat | 2,00 | |
| Glycerin | 3,00 | |
| Magnesiumaluminiumsilikat | 0,10 | |
| Bentonit | 0,10 | |
| Polyacrylsäure | 0,30 | |
| Hydroxypropylcellulose | 0,10 | |
| Parfüm, Konservierungsmittel, Farbstoffe usw. | q.s. | |
| Natriumhydroxid | q.s. | |

(fortgesetzt)

| | | |
|---|---|---|
| Wasser | ad 100,00 | |
| pH-Wert eingestellt auf 6,0-7,5 | | |
| Emulsion VI | | 35 |
| Gas (Argon) | | 65 |

Vordispergierung der anorganischen Gelbildner und Quellung der Hydrokolloide unter Rühren in der Wasserphase. Vereinigung der auf 78˚C aufgeheizten Fettphase mit der auf 75˚C aufgeheizten Wasserphase. Homogenisierung mittels einer Zahnkranzdispergiermaschine (Rotor-Stator-Prinzip) bei 65˚C. 45 min Rühren im Becomix unter Begasung mit Argon bei 1 bar unter Kühlung auf 30˚C. Zugabe der Additive bei 30˚C (Parfüm, Wirkstoffe). Homogenisierung mittels einer Zahnkranzdispergiermaschine (Rotor-Stator-Prinzip) bei 23˚C.

**Beispiel 7 (schaumförmige Sonnenschutz-Creme):**

| Emulsion VII | Gew.-% | Vol.-% |
|---|---|---|
| Stearinsäure | 1,00 | |
| Cetylstearylalkohol | 4,00 | |
| Myristylalkohol | 1,00 | |
| PEG-20-Stearat | 1,00 | |
| Xanthangummi | 0,10 | |
| Polyacrylsäure | 0,30 | |
| Hectorit | 0,20 | |
| Quaternium-18-Hectorit | 0.10 | |
| Caprylsäure/Caprinsäuretriglyceride | 2,00 | |
| Paraffinöl | 15,50 | |
| Dimethicon | 0,50 | |
| Octylisostearat | 5,00 | |
| Glycerin | 3,00 | |
| Octylmethoxycinnamat | 4,00 | |
| Butylmethoxydibenzoylmethan | 3,00 | |
| Ethylhexyl Triazon | 3,00 | |
| BHT | 0,02 | |
| Disodium EDTA | 0,10 | |
| Parfüm, Konservierungsmittel, | q.s. | |
| Farbstoffe, usw. | q.s. | |
| Kaliumhydroxid | q.s | |
| Wasser | ad 100,00 | |
| pH-Wert eingestellt auf 5,0-6,0 | | |
| Emulsion VII | | 35 |
| Gas (Helium) | | 65 |

Vordispergierung des anorganischen Gelbildners (Hectorite) und Quellung der Hydrokolloide unter Rühren in der Wasserphase. Vordispergierung des Quaternium-18-Hectorits in der heißen Fettphase. Vereinigung der auf 78˚C aufgeheizten Fett-/Lichtschutzfilterphase mit der auf 75˚C aufgeheizten Wasser-/Lichtschutzfilterphase. Homogenisierung mittels einer Zahnkranzdispergiermaschine (Rotor-Stator-Prinzip) bei 65˚C. 45 min Rühren im Becomix unter Begasung mit Helium bei 1 bar unter Kühlung auf 30˚C. Zugabe der Additive bei 30˚C (Parfüm). Homogenisierung mittels einer Zahnkranzdispergiermaschine (Rotor-Stator-Prinzip) bei 23˚C.

**Patentansprüche**

1. Selbstschäumende und/oder schaumförmige kosmetische oder dermatologische Zubereitungen, welche

   I. ein Emulgatorsystem, welches aus

   A. mindestens einem Emulgator A, gewählt aus der Gruppe der ganz-, teil- oder nichtneutralisierten, verzweigten und/oder unverzweigten, gesättigten und/oder ungesättigten Fettsäuren mit einer Kettenlänge von 10 bis 40 Kohlenstoffatomen,
   B. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und
   C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,

   besteht,
   II. bis zu 30 Gew.-% - bezogen auf das Gesamtgewicht der Zubereitung - einer Lipidphase,
   III. 1 bis 90 Vol.-%, bezogen auf das Gesamtvolumen der Zubereitung, mindestens eines Gases, gewählt aus der Gruppe Luft, Sauerstoff, Stickstoff, Helium, Argon, Lachgas ($N_2O$) und Kohlendioxid ($CO_2$)
   IV. 0,01 bis 10% ein oder mehrere Gelbildner ausgewählt aus der Gruppe der anorganischen Verdickungsmitteln,
   V. eine oder mehrere Substanzen, gewählt aus der Gruppe der organischen Hydrokolloide.

   enthalten.

2. Zubereitung nach Anspruch 1, **dadurch gekennzeichnet, daß** die Gewichtsverhältnisse von Emulgator A zu Emulgator B zu Coemulgator C (A : B : C) wie a : b : c gewählt wird, wobei a, b und c unabhängig voneinander rationale Zahlen von 1 bis 5, bevorzugt von 1 bis 3 darstellen.

3. Zubereitung nach einem der vorhergehenden Ansprüche, **dadurch gekennzeichnet, daß** die Gewichtsverhältnisse von Emulgator A zu Emulgator B zu Coemulgator C (A : B : C) wie etwa 1 : 1 : 1 gewählt wird

4. Zubereitung nach einem der vorhergehenden Ansprüche, **dadurch gekennzeichnet, daß** die Gesamtmenge der Substanzen gemäß A., B. und C. aus dem Bereich von 2 bis 20 Gew.-%, bezogen auf das Gesamtgewicht der Formulierung, gewählt werden.

5. Zubereitung nach einem der vorhergehenden Ansprüche, **dadurch gekennzeichnet, daß** sie weitere Emulgatoren, gewählt aus der Gruppe der hydrophilen Emulgatoren, insbesondere Mono-, Di-, Trifettsäureestern des Sorbitols, enthalten.

6. Zubereitung nach Anspruch 5, **dadurch gekennzeichnet, daß** die Gesamtmenge der weiteren Emulgatoren kleiner als 5 Gew.-%, bezogen auf das Gesamtgewicht der Formulierung, gewählt wird.

7. Zubereitung nach einem der vorhergehenden Ansprüche, **dadurch gekennzeichnet, daß** die Gesamtmenge an einem oder mehreren Hydrokolloiden in den fertigen kosmetischen oder dermatologischen Zubereitungen kleiner als 5% Gew. %, bevorzugt zwischen 0,1 und 1,0 Gew.-%, bezogen auf das Gesamtgewicht der Zubereitungen, gewählt wird.

8. Zubereitung nach einem der vorhergehenden Ansprüche, **dadurch gekennzeichnet, daß** der Volumenanteil des oder der Gase von 10 bis 80 Vol.-%, bezogen auf das Gesamtvolumen der Zubereitung, gewählt wird.

9. Zubereitung nach einem der vorhergehenden Ansprüche, **dadurch gekennzeichnet, daß** als Gas Kohlendioxid gewählt wird.

10. Zubereitung nach einem der vorhergehenden Ansprüche, **dadurch gekennzeichnet, daß** sie eine oder mehrere Substanzen, gewählt aus der Gruppe der Moisturizer, enthält.

11. Verwendung selbstschäumender und/oder schaumförmiger kosmetischer oder dermatologischer Zubereitungen, welche

I. ein Emulgatorsystem, welches aus

A. mindestens einem Emulgator A, gewählt aus der Gruppe der ganz-, teil- oder nichtneutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,

besteht,
II. bis zu 30 Gew.-% - bezogen auf das Gesamtgewicht der Zubereitung - einer Lipidphase,
IV. 0,01-5 bis 10% Gew. % eines oder mehrerer Gelbildner ausgewählt aus der Gruppe der anorganischen Verdickungsmitteln,
V. eine oder mehrere Substanzen, gewählt aus der Gruppe der organischen Hydrokolloide,

enthalten, als kosmetische oder dermatologische Grundlagen für gasförmige Wirkstoffe.

**12.** Zubereitung oder Verwendung nach einem der vorhergehenden Ansprüche, **dadurch gekennzeichnet, daß** das oder die anorganischen Verdickungsmittel gewählt werden aus der Gruppe der modifizierten oder unmodifizierten, natürlich vorkommenden oder synthetischen Schichtsilikate.

**13.** Zubereitung oder Verwendung nach einem der vorhergehenden Ansprüche, **dadurch gekennzeichnet, daß** das oder die anorganischen Verdickungsmittel gewählt werden aus der Gruppe der Magnesium-Aluminium-Silikate, Magnesiumsilikate sowie Natrium-Magnesium-Silikate, insbesonsere der Montmorillonite umfassend Bentonite, Hectorite sowie deren gegebenenfalls organisch modifizierten Derivate wie Quaternium-18 Bentonit, Quaternium-18 Hectorite, Stearalkonium Bentonite bzw. Stearalkonium Hectorite

**14.** Zubereitung oder Verwendung nach einem der vorhergehenden Ansprüche, **dadurch gekennzeichnet, daß** das oder die organischen Hydrokolloide gewählt werden aus der Gruppe der

a) organischen, vollsynthetischen Verbindungen der Polyacrylsäuren.
b) der Co- und Crosspolymere der Polyacrylsäurederivate wie Polymethacrylate, Acrylat Copolymere, Alkylacrylatcopolymere, Acrylamide, Alkylacrylatcrosspolymere, Acrylonitrogen Copolymere,
c) Co- und Crosspolymere wie Ammoniumdimethyltauramid/Vinylformamid Copolymer
d) Copolymere/Crosspolymere umfassend Acryloyldimethyltaurate
e) der hydrophilen Gummen und deren hydrophilen Derivate umfassend anionische wie Agar-Agar, Alginsäure, Carrageen, Gelatine, Gummiarabikum, Pektin, Tragant,
nichtionogene wie Galaktomannane (Guar-Gummi), Johannisbrotkernmehl, Xanthangummi, Polyvinylalkohol, Polyvinylpyrrolidon, Propylenglykolalginat, Stärke)
f) der Cellulose und/oder mikrokristalline Cellulose sowie besonders bevorzugt Cellulose-Derivate umfassend akylmodifizierte Cellulose-Derivate (wie Methylcellulose) sowie Alkylhydroxycellulose (wie Hydroxymethylcellulose, Hydroxyethylcellulose) und beliebige Mischungen daraus.

## Claims

**1.** Self-foaming and/or foam-like cosmetic or dermatological preparations which comprise

I. an emulsifier system which consists of

A. at least one emulsifier A chosen from the group of wholly neutralized, partially neutralized or unneutralized branched and/or unbranched, saturated and/or unsaturated fatty acids having a chain length of from 10 to 40 carbon atoms,
B. at least one emulsifier B chosen from the group of polyethoxylated fatty acid esters having a chain length of from 10 to 40 carbon atoms and a degree of ethoxylation of from 5 to 100 and
C. at least one coemulsifier C chosen from the group of saturated and/or unsaturated, branched and/or unbranched fatty alcohols having a chain length of from 10 to 40 carbon atoms,

II. up to 30% by weight - based on the total weight of the preparation - of a lipid phase,
III. 1 to 90% by volume, based on the total volume of the preparation, of at least one gas chosen from the group consisting of air, oxygen, nitrogen, helium, argon, nitrous oxide ($N_2O$) and carbon dioxide ($CO_2$)
IV. 0.01 to 10% of one or more gelling agents chosen from the group of inorganic thickeners,
V. one or more substances chosen from the group of organic hydrocolloids.

**2.** Preparation according to Claim 1, **characterized in that** the weight ratios of emulsifier A to emulsifier B to coemulsifier C (A : B : C) is chosen as a : b : c, where a, b and c, independently of one another, are rational numbers from 1 to 5, preferably from 1 to 3.

**3.** Preparation according to any of the preceding claims, **characterized in that** the weight ratios of emulsifier A to emulsifier B to coemulsifier C (A : B : C) is chosen as, for example, 1 : 1 : 1.

**4.** Preparation according to any of the preceding claims, **characterized in that** the total amount of the substances according to A., B. and C. are chosen from the range from 2 to 20% by weight, based on the total weight of the formulation.

**5.** Preparation according to any of the preceding claims, **characterized in that** it comprises further emulsifiers chosen from the group of hydrophilic emulsifiers, in particular mono-, di-, tri-fatty acid esters of sorbitol.

**6.** Preparation according to Claim 5, **characterized in that** the total amount of the further emulsifiers is chosen to be less than 5% by weight, based on the total weight of the formulation.

**7.** Preparation according to any of the preceding claims, **characterized in that** the total amount of one or more hydrocolloids in the finished cosmetic or dermatological preparations is chosen to be less than 5% by weight, preferably between 0.1 and 1.0% by weight, based on the total weight of the preparations.

**8.** Preparation according to any of the preceding claims, **characterized in that** the proportion by volume of the gas (es) is chosen from 10 to 80% by volume, based on the total volume of the preparation.

**9.** Preparation according to any of the preceding claims, **characterized in that** the gas chosen is carbon dioxide.

**10.** Preparation according to any of the preceding claims, **characterized in that** it comprises one or more substances chosen from the group of moisturizers.

**11.** Use of self-foaming and/or foam-like cosmetic or dermatological preparations which comprise

I. an emulsifier system which consists of

A. at least one emulsifier A chosen from the group of wholly neutralized, partially neutralized or unneutralized branched and/or unbranched, saturated and/or unsaturated fatty acids having a chain length of from 10 to 40 carbon atoms,
B. at least one emulsifier B chosen from the group of polyethoxylated fatty acid esters having a chain length of from 10 to 40 carbon atoms and a degree of ethoxylation of from 5 to 100 and
C. at least one coemulsifier C chosen from the group of saturated and/or unsaturated, branched and/or unbranched fatty alcohols having a chain length of from 10 to 40 carbon atoms,

II. up to 30% by weight - based on the total weight of the preparation - of a lipid phase,
IV. 0.01-5 to 10% by weight of one or more gelling agents chosen from the group of inorganic thickeners,
V. one or more substances chosen from the group of organic hydrocolloids as cosmetic or dermatological bases for gaseous active ingredients.

**12.** Preparation or use according to any of the preceding claims, **characterized in that** the inorganic thickener or thickeners are chosen from the group of modified or unmodified, naturally occurring or synthetic phyllosilicates.

**13.** Preparation or use according to any of the preceding claims, **characterized in that** the inorganic thickener or thickeners are chosen from the group of magnesium aluminum silicates, magnesium silicates, and sodium magnesium silicates, in particular the montmorillonites comprising bentonites, hectorites and their optionally organically

modified derivatives, such as quaternium-18 bentonite, quaternium-18 hectorites, stearalkonium bentonites and stearalkonium hectorites.

**14.** Preparation or use according to any of the preceding claims, **characterized in that** the organic hydrocolloid(s) is/are chosen from the group of

a) organic, completely synthetic compounds of polyacrylic acids,
b) copolymers and crosspolymers of polyacrylic acid derivatives, such as polymethacrylates, acrylate copolymers, alkyl acrylate copolymers, acrylamides, alkyl acrylate crosspolymers, acrylonitrogen copolymers,
c) copolymers and crosspolymers, such as ammonium dimethyltauramide/vinylformamide copolymer
d) copolymers/crosspolymers comprising acryloyldimethyltaurates
e) hydrophilic gums and hydrophilic derivatives thereof including
anionic ones, such as agar agar, alginic acid, carrageen, gelatin, gum arabic, pectin, tragacanth,
nonionogenic ones, such as galactomannans (guar gum), carob seed grain, xanthan gum, polyvinyl alcohol, polyvinylpyrrolidone, propylene glycol alginate, starch)
f) cellulose and/or microcrystalline cellulose, and particularly preferably cellulose derivatives including alkyl-modified cellulose derivates (such as methylcellulose) and alkylhydroxycellulose (such as hydroxymethylcellulose, hydroxyethylcellulose) and any mixtures thereof.

## Revendications

**1.** Préparations cosmétiques ou dermatologiques auto-moussantes et/ou moussantes, qui contiennent

I. un système émulsionnant, qui est constitué

A. d'au moins un émulsionnant A choisi dans le groupe des acides gras complètement, partiellement ou non neutralisés, ramifiés et/ou non ramifiés, saturés et/ou insaturés, avec une longueur de chaîne de 10 à 40 atomes de carbone,
B. d'au moins un émulsionnant B choisi dans le groupe des esters d'acides gras polyéthoxylés ayant une longueur de chaîne de 10 à 40 atomes de carbone et un degré d'éthoxylation de 5 à 100, et
C. d'au moins un co-émulsionnant C choisi dans le groupe des alcools gras saturés et/ou insaturés, ramifiés et/ou non ramifiés, avec une longueur de chaîne de 10 à 40 atomes de carbone,

II. jusqu'à 30% en poids par rapport au poids total de la préparation d'une phase de lipide,
III. 1 à 90% en volume, par rapport au volume total de la préparation, d'au moins un gaz choisi dans le groupe de l'air, de l'oxygène, de l'azote, de l'hélium, de l'argon, du gaz hilarant ($N_2O$) et du dioxyde de carbone ($CO_2$),
IV. 0,01 à 10% en poids d'un ou plusieurs gélifiants choisis dans le groupe des agents épaississants inorganiques, et
V. d'une ou plusieurs substances choisies dans le groupe des hydrocolloïdes organiques.

**2.** Préparation selon la revendication 1, **caractérisée en ce que** les rapports pondéraux de l'émulsionnant A à l'émulsionnant B au co-émulsionnant C (A:B:C) sont choisis tels que a:b:c, de sorte que a, b et c représentent, indépendamment l'un de l'autre, des chiffres rationnels de 1 à 5, de préférence de 1 à 3.

**3.** Préparation selon l'une quelconque des revendications précédentes, **caractérisée en ce que** le rapport pondéral de l'émulsionnant A à l'émulsionnant B au co-émulsionnant C (A:B:C) est choisi à environ 1:1:1.

**4.** Préparation selon l'une quelconque des revendications précédentes, **caractérisée en ce que** les quantités totales des substances selon A, B et C sont choisies dans la plage de 2 à 20% en poids par rapport au poids total de la formulation.

**5.** Préparation selon l'une quelconque des revendications précédentes, **caractérisée en ce qu'**elle contient d'autres émulsionnants choisis dans le groupe des émulsionnants hydrophiles, en particulier des esters des mono-, di- et triacides gras du sorbitol.

**6.** Préparation selon la revendication 5, **caractérisée en ce que** la quantité totale des autres émulsionnants est choisie inférieure à 5% en poids par rapport au poids total de la formulation.

**7.** Préparation selon l'une quelconque des revendications précédentes, **caractérisée en ce que** la quantité totale d'un ou plusieurs hydrocolloïdes dans les préparations cosmétiques ou dermatologiques finies est choisie inférieure à 5% en poids, de préférence entre 0,1 et 1,0% en poids, par rapport au poids total des préparations.

**8.** Préparation selon l'une quelconque des revendications précédentes, **caractérisée en ce que** la fraction volumique du ou des gaz est choisie à raison de 10 à 80% en volume par rapport au volume total de la préparation.

**9.** Préparation selon l'une quelconque des revendications précédentes, **caractérisée en ce que** l'on choisit comme gaz du dioxyde de carbone.

**10.** Préparation selon l'une quelconque des revendications précédentes, **caractérisée en ce qu'**elle contient une ou plusieurs substances choisies dans le groupe des humectants.

**11.** Utilisation de préparations cosmétiques ou dermatologiques auto-moussantes et/ou moussantes, qui contiennent

I. un système émulsionnant, qui est constitué

A. d'au moins un émulsionnant A choisi dans le groupe des acides gras complètement, partiellement ou non neutralisés, ramifiés et/ou non ramifiés, saturés et/ou insaturés, avec une longueur de chaîne de 10 à 40 atomes de carbone,
B. d'au moins un émulsionnant B choisi dans le groupe des esters d'acides gras polyéthoxylés ayant une longueur de chaîne de 10 à 40 atomes de carbone et un degré d'éthoxylation de 5 à 100, et
C. d'au moins un co-émulsionnant C choisi dans le groupe des alcools gras saturés et/ou insaturés, ramifiés et/ou non ramifiés, avec une longueur de chaîne de 10 à 40 atomes de carbone,

II. jusqu'à 30% en poids par rapport au poids total de la préparation d'une phase de lipide,
IV. 0,01 à 10% en poids d'un ou plusieurs gélifiants choisis dans le groupe des agents épaississants inorganiques, et
V. d'une ou plusieurs substances choisies dans le groupe des hydrocolloïdes organiques,

comme bases cosmétiques ou dermatologiques pour des principes actifs formateurs de gaz.

**12.** Préparation ou utilisation selon l'une quelconque des revendications précédentes, **caractérisée en ce que** l'on choisit le ou les agents épaississants inorganiques dans le groupe des silicates stratifiés naturels ou synthétiques modifiés ou non modifiés.

**13.** Préparation ou utilisation selon l'une quelconque des revendications précédentes, **caractérisée en ce que** l'on choisit le ou les agents épaississants inorganiques dans le groupe des silicates de magnésium et d'aluminium, des silicates de magnésium ainsi que des silicates de sodium et de magnésium, en particulier la montmorillonite comprenant la bentonite, l'hectorite ainsi que leurs dérivés éventuellement organiquement modifiés, tels que la bentonite de quaternium-18, l'hectorite de quaternium-18, la bentonite de stéaralkonium ou l'hectorite de stéaralkonium.

**14.** Préparation ou utilisation selon l'une quelconque des revendications précédentes, **caractérisée en ce que** l'hydrocolloïde organique ou les hydrocolloïdes organiques est choisi ou sont choisis dans le groupe suivant :

a) des composés organiques entièrement synthétiques d'acides polyacryliques,
b) les copolymères et les polymères croisés de dérivés de l'acide polyacrylique, tels que des polyméthacrylates, des copolymères d'acrylate, des copolymères d'acrylate d'alkyle, des acrylamides, des polymères croisés d'acrylate d'alkyle, des copolymères acrylo-azotés,
c) des copolymères et des polymères croisés tels qu'un copolymère diméthyltauramide d'ammonium/vinylformamide,
d) des copolymères/polymères croisés comprenant des acryloyldiméthyltaurates,
e) les gommes hydrophiles et leurs dérivés hydrophiles comprenant
des dérivés anioniques tels que la gellose, l'acide alginique, la mousse perlée d'Irlande, la gelatine, la gomme arabique, la pectine, la gomme adragante,
des dérivés non ionogènes tels que des galactotomannanes (gomme de guar), la farine de graines de caroube, la gomme de xanthane, l'alcool polyvinylique, la polyvinylpyrrolidone, l'alginate de propylèneglycol, des amidons,
f) la cellulose et/ou la cellulose microcristalline et particulièrement préférablement des dérivés de la cellulose

comprenant des dérivés de la cellulose alkyl-modifiés (tels que la méthylcellulose) ainsi que des alkylhydroxycelluloses (tels que l'hydroxyméthylcellulose, l'hydroxyéthylcellulose) et leurs mélanges quelconques.